(19) **Europäisches Patentamt / European Patent Office / Office européen des brevets**

(11) **EP 3 385 757 B1**

(12) **FASCICULE DE BREVET EUROPEEN**

(45) Date de publication et mention
de la délivrance du brevet:
**09.10.2019 Bulletin 2019/41**

(51) Int Cl.:
**G01T 1/36** (2006.01)      **A61B 6/00** (2006.01)
**G01T 1/29** (2006.01)

(21) Numéro de dépôt: **18165796.6**

(22) Date de dépôt: **04.04.2018**

(54) **PROCÉDÉ DE TRAITEMENT D'UN SPECTRE D'UN RAYONNEMENT MESURÉ PAR UN DÉTECTEUR**

**VERARBEITUNGSVERFAHREN EINES MIT EINEM DETEKTOR GEMESSENEN SPEKTRUMS EINER STRAHLUNG**

**METHOD FOR TREATING A RADIATION SPECTRUM MEASURED BY A DETECTOR**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.04.2017 FR 1753063**

(43) Date de publication de la demande:
**10.10.2018 Bulletin 2018/41**

(73) Titulaire: **COMMISSARIAT À L'ÉNERGIE ATOMIQUE ET AUX ÉNERGIES ALTERNATIVES**
**75015 Paris (FR)**

(72) Inventeurs:
• **SOSSIN, Artur**
  **69372 LYON Cedex 08 (FR)**
• **REBUFFEL, Veronique**
  **38700 CORENC (FR)**
• **TABARY, Joachim**
  **38100 GRENOBLE (FR)**

(74) Mandataire: **GIE Innovation Competence Group**
**310, avenue Berthelot**
**69372 Lyon Cedex 08 (FR)**

(56) Documents cités:
**FR-A1- 3 024 235      JP-A- H04 159 879**
**US-A1- 2009 238 324**

**Description**

**DOMAINE TECHNIQUE**

Le domaine technique de l'invention est le traitement de spectres d'un rayonnement transmis par un objet, ce dernier étant irradié par une source de rayonnement ionisant, en particulier un rayonnement X. L'objectif de l'invention est d'obtenir une pluralité de spectres à l'aide d'un détecteur pixellisé, et d'effectuer une correction de tout ou partie des spectres, de façon à limiter l'influence d'une composante représentative d'un rayonnement diffusé. Les applications se trouvent dans l'imagerie médicale, dans le contrôle non destructif ou dans le domaine de la sécurité des personnes.

**ART ANTERIEUR**

Le contrôle d'objets par rayonnement X, dans le domaine médical ou industriel, est très répandu. Les procédés existants consistent à disposer un objet entre une source de rayonnement et un détecteur, puis à irradier l'objet à l'aide de la source. Le détecteur forme alors une image, généralement en deux dimensions, du rayonnement transmis par l'objet. Cette image est représentative de l'atténuation, par l'objet, du rayonnement émis par la source.

Le rayonnement transmis par l'objet comporte généralement une composante résultant de la diffusion, par l'objet, du rayonnement émis par la source. Elle est d'autant plus significative que l'énergie du rayonnement est faible et/ou que l'objet est constitué de matériaux dont le numéro atomique est élevé. Cette composante, appelée communément rayonnement de diffusion, perturbe l'interprétation des images, car elle n'est qu'indirectement liée à l'atténuation par l'objet. De plus, alors que le rayonnement non diffusé, dit rayonnement primaire, se propage entre la source et le détecteur selon une trajectoire rectiligne, le rayonnement diffusé a pour origine un point quelconque de l'objet, et sa trajectoire, depuis ce point origine, est distribuée selon différents angles. On recherche donc à estimer cette composante de diffusion, de façon à l'extraire du signal mesuré par le détecteur, préalablement au traitement des images en vue de leur interprétation.

Des outils de simulation numérique permettent de déterminer le rayonnement primaire et le rayonnement de diffusion ayant traversé un objet analysé. Un exemple en est donné dans la publication Sossin A. "Fast scattering simulation tool for multi-energy x-ray imaging" Nuclear Instruments and Methods in Physics Research, A 802 (2015) 60-66. De tels outils facilitent le développement de systèmes de radiographie ou de tomographie.

Dans le domaine de l'imagerie médicale, de nombreuses méthodes ont été développées pour tenter d'estimer et de réduire la part du rayonnement diffusé, de façon à obtenir une image essentiellement représentative du rayonnement non diffusé, dit rayonnement primaire, se propageant entre la source et l'objet selon une direction rectiligne.

Par exemple, la publication Zhu L, « Scatter Correction Method for X-ray CT Using Primary Modulation : Theory and Preliminary Results », IEEE Transactions on Médical Imaging, Vol. 25, N° 12, dec 2006, décrit un procédé consistant à interposer un masque amovible, prenant la forme d'une grille, entre une source de rayons X et un objet. Cette publication est basée sur le fait qu'un tel masque engendre une modulation spatiale significative du rayonnement primaire, dans des fréquences spatiales élevées, tandis que son influence sur le rayonnement diffusé, dans les fréquences spatiales faibles, est moins importante. Ainsi, dans un espace fréquentiel, il est possible d'établir une discrimination entre le rayonnement primaire et le rayonnement diffusé.

Par ailleurs, la publication Ning R, «X-ray Scatter Correction Algorithm for Cone Beam CT-Imaging », Med.Phys. 31 (5), May 2004, décrit également un procédé consistant à interposer un masque amovible entre une source de rayons X et un objet. Le masque produit une atténuation telle que lorsqu'il est disposé entre la source et l'objet, le rayonnement mesuré par le détecteur est considéré comme représentatif du seul rayonnement diffusé. Autrement dit, le masque produit une atténuation, considérée comme totale, du rayonnement primaire.

Le principe d'un masque amovible est également décrit dans la publication Yang K, "A breast-specific, negligible-dose scatter correction technique for dedicated cone-beam breast CT : a physics-based approach to improve Hounsfield Unit accuracy", Phys.Med.Biol. 59 (2014) 6487-6505. Il est également décrit dans le document FR3024235.

Les détecteurs actuels permettent l'obtention d'images en deux dimensions dont la qualité ne cesse de s'améliorer. Une évolution récente est l'apparition de détecteurs permettant l'acquisition d'images spectrales, c'est-à-dire d'images selon différentes bandes d'énergie. Ces détecteurs, fréquemment basés sur des détecteurs semi-conducteurs ayant une fonction spectrométrique, ajoutent une dimension spectrale aux données acquises, ces dernières étant généralement obtenues selon deux dimensions. Il est alors possible d'obtenir une image du rayonnement transmis par l'objet selon différentes bandes d'énergie.

Or, les méthodes précitées ne concernent pas l'imagerie spectrale. Les inventeurs proposent une solution alternative, basée sur l'imagerie spectrale, permettant une correction efficace du rayonnement détecté par le détecteur, de façon à en extraire le rayonnement primaire, et cela simultanément dans plusieurs bandes d'énergie. Les essais présentés à la fin de la description témoignent de l'efficacité de la méthode.

## EXPOSE DE L'INVENTION

Les objets de l'invention sont revendiqués dans les revendications indépendantes 1, 2, 15 et 16.

Un premier objet de l'invention est un procédé de correction d'un spectre d'un rayonnement électromagnétique ionisant transmis par un objet,

- l'objet étant disposé entre une source d'irradiation et un détecteur, la source d'irradiation étant apte à émettre un rayonnement électromagnétique ionisant, dit rayonnement incident, vers ledit objet ;
- le détecteur comportant des pixels, chaque pixel étant configuré pour détecter un rayonnement transmis par l'objet vers le détecteur, et à en acquérir un spectre, le rayonnement transmis comportant un rayonnement diffusé, causé par la diffusion du rayonnement incident dans l'objet, et un rayonnement primaire ;
- un masque étant interposé entre la source et l'objet, le masque comportant des éléments absorbants, aptes à atténuer une partie dudit rayonnement incident selon une atténuation, la projection de chaque élément absorbant sur le détecteur formant une zone d'ombre, de telle sorte que le détecteur comporte une pluralité de zones d'ombre espacées les unes des autres, chaque zone d'ombre comportant au moins un pixel ;

le procédé comportant les étapes suivantes :

a) irradiation de l'objet et acquisition, par les pixels du détecteur, d'un spectre du rayonnement transmis par l'objet ;

b) sélection d'une région d'analyse, la région d'analyse comportant au moins un pixel appartenant à une zone d'ombre, dite zone d'ombre d'analyse, et un pixel situé à l'extérieur de ladite zone d'ombre d'analyse ;

c) prise en compte d'un spectre, dit spectre de diffusion, représentant le rayonnement diffusé par l'objet atteignant la région d'analyse ;

d) pour au moins un pixel de la région d'analyse, situé dans la zone d'ombre d'analyse, à partir de l'atténuation du masque et du spectre de diffusion pris en compte lors de l'étape c), estimation d'un spectre du rayonnement primaire transmis par l'objet, en l'absence de masque ;

e) pour au moins un pixel de la région d'analyse, situé en dehors de la zone d'ombre d'analyse, à partir du spectre de diffusion pris en compte de l'étape c), détermination d'un spectre du rayonnement primaire transmis par l'objet ;

f) détermination d'un écart entre les spectres du rayonnement primaire déterminés lors des étapes d) et e), l'écart dépendant du spectre de diffusion pris en compte lors de l'étape c) ;

g) répétition des étapes c) à f) en prenant en compte, lors de l'étape c), différents spectres de diffusion, de façon à déterminer un spectre de diffusion, pris en compte lors de l'étape c), minimisant l'écart déterminé lors de l'étape f), pour la région d'analyse sélectionnée lors de l'étape b);

h) détermination du spectre de diffusion minimisant l'écart déterminé lors de chaque étape f), le spectre de diffusion ainsi déterminé étant associé à la région d'analyse sélectionnée;

i) répétition des étapes b) à h) en sélectionnant différentes régions d'analyse, de façon à déterminer autant de spectres de diffusion que de régions d'analyse;

j) correction des spectres acquis lors de l'étape a) à partir des spectres de diffusion résultant de l'étape i).

L'étape j) peut notamment comporter, à partir des spectres de diffusion résultant de l'étape i), l'association d'un spectre de diffusion à chaque pixel et la correction de spectres acquis lors de l'étape a), par au moins un pixel, en fonction du spectre de diffusion associé audit pixel.

Pour chaque pixel du détecteur, le spectre corrigé constitue alors une estimation du spectre d'un rayonnement primaire transmis par l'objet. Il représente plus fidèlement les propriétés d'atténuation du rayonnement ionisant par l'objet.

Par zone d'ombre d'analyse, on entend une zone d'ombre située partiellement ou totalement dans la région d'analyse.

Les étapes d) et e) peuvent être effectuées en considérant tout ou partie des pixels respectivement situés à l'intérieur et à l'extérieur de chaque zone d'ombre d'analyse.

Selon une variante, le procédé comporte les étapes suivantes a) à c) et f) à j) telles que précédemment décrites, les étapes d) et e) étant :

d) pour chaque pixel de la région d'analyse situé en dehors de la zone d'ombre d'analyse, à partir de l'atténuation du masque du spectre de diffusion pris en compte de l'étape c), estimation d'un spectre du rayonnement primaire transmis par l'objet en présence d'un élément absorbant du masque entre la source d'irradiation et ledit pixel;

e) pour chaque pixel de la région d'analyse et situé dans la zone d'ombre d'analyse, à partir du spectre de diffusion pris en compte lors de l'étape c), estimation d'un spectre du rayonnement primaire transmis par l'objet.

Le principe de cette méthode repose sur une estimation du spectre de diffusion minimisant un écart entre les spectres du rayonnement primaire déterminés respectivement à l'extérieur et à l'intérieur de chaque zone d'ombre d'analyse. Deux alternatives se présentent : selon une première alternative, qui correspond au mode de réalisation préféré, on compare un ou plusieurs spectres primaires mesurés à l'extérieur d'une zone d'ombre d'analyse avec une estimation d'au moins un spectre primaire qui serait mesuré, à l'intérieur de la zone d'ombre d'analyse, en l'absence de masque. Selon une deuxième alternative, on compare un ou plusieurs spectres primaires mesurés à l'intérieur d'une zone d'ombre d'analyse avec une estimation d'au moins un spectre primaire qui serait mesuré, à l'extérieur de la zone d'ombre d'analyse, en présence de masque.

L'étape j) peut comporter l'association, à tout ou partie des pixels du détecteur, d'un spectre de diffusion obtenu à partir des différents spectres de diffusion résultant de l'étape i).

Selon un mode de réalisation, l'étape d) comporte les sous-étapes suivantes :

di) soustraction du spectre de diffusion pris en compte lors de l'étape c) au spectre mesuré, pour le pixel, lors de l'étape a) ;
dii) application d'une matrice de passage au spectre résultant de la sous-étape di), la matrice de passage prenant en compte l'atténuation d'un élément absorbant du masque.

Lors de l'étape f), l'indicateur d'écart peut être calculé en comparant, par exemple sous la forme d'une soustraction ou d'un ratio, des spectres résultant respectivement de l'étape d) et de l'étape e), pour une même région d'analyse, ou de leurs valeurs moyennes respectives, ou de leurs valeurs médianes respectives.

La matrice de passage peut notamment être obtenue par simulation numérique ou au cours d'une phase de calibration, au cours de laquelle l'objet est remplacé par un objet de calibration comportant les étapes suivantes :

cal-i) formation d'une matrice diagonale, chaque terme de la diagonale représentant une atténuation par le masque dans une plage d'énergie ;

irradiation de l'objet de calibration et acquisition, par un pixel du détecteur de calibration du détecteur, dit pixel de calibration,
d'un spectre du rayonnement transmis par l'objet de calibration lorsque le masque est interposé entre la source et le détecteur;

cal-ii) d'un spectre du rayonnement transmis par l'objet de calibration en l'absence de masque interposé entre la source et le détecteur;
cal-iii) définition d'une matrice de passage en appliquant une fonction de déformation à la matrice diagonale, la fonction de déformation dépendant de paramètres ;
cal-iv) application de la matrice de passage au spectre du rayonnement transmis par l'objet de calibration en présence du masque ;
cal-v) réitération des étapes cal-ii) à cal-iv) pour différents objets de calibration ;
cal-vi) obtention des paramètres de la fonction minimisant un écart entre les spectres résultant de l'étape cal-iv) à chaque spectre du rayonnement transmis par l'objet de calibration en l'absence du masque interposé entre la source et le détecteur.

Selon une variante, l'étape cal iv) comporte l'application de la matrice de passage au spectre du rayonnement transmis par l'objet de calibration en l'absence du masque et l'étape cal-vi) comporte l'obtention des paramètres de la fonction minimisant un écart entre les spectres résultant de l'étape cal-iv) à chaque spectre du rayonnement transmis par l'objet de calibration en présence du masque interposé entre la source et le détecteur.

L'étape j) peut comporter pour chaque pixel situé à l'intérieur d'une zone d'ombre, les sous-étapes suivantes :

ji) détermination d'un spectre primaire, dit spectre primaire atténué, à partir du spectre acquis, par le pixel, lors de l'étape a) en fonction du spectre de diffusion associé audit pixel lors de l'étape j);
jii) correction du spectre résultant de la sous-étape ji) en prenant en compte l'atténuation du masque, pour estimer un spectre primaire en l'absence de masque.

L'étape j) peut comporter, pour chaque pixel situé à l'extérieur d'une zone d'ombre, une soustraction du spectre de diffusion associé audit pixel lors de l'étape j) au spectre acquis par le pixel, lors de l'étape a).

Le procédé peut comporter, suite à l'étape i), un lissage entre les spectres de diffusion respectivement associés à des pixels adjacents.

Selon un mode de réalisation, à chaque région d'analyse est associé un pixel, le procédé étant mis en oeuvre sur les régions d'analyse associées à tout ou partie des pixels du détecteur.

Chaque élément absorbant peut être apte à atténuer entre 5 % et 80 % du rayonnement auquel il est exposé. Le masque peut s'étendre selon une surface, plane ou curviligne, chaque élément absorbant est distant d'un autre élément absorbant d'une distance inférieure à 1 cm. Le masque est de préférence interposé entre la source d'irradiation et l'objet.

Les étapes a) à j) peuvent être complétées par les étapes suivantes :

k) sélection d'au moins une énergie ou une plage d'énergie;
l) réalisation d'une image, dont chaque pixel comprend une donnée obtenue à partir d'un spectre corrigé, associé à un pixel du détecteur, à ladite énergie ou dans ladite plage d'énergie. Lors de cette étape, chaque pixel de l'image peut comporter une information relative à une intégrale ou à une moyenne dudit spectre corrigé dans ladite plage d'énergie.

Un deuxième objet de l'invention est un support d'enregistrement d'informations, comportant des instructions pour l'exécution des étapes b) à j) du procédé selon le premier objet de l'invention, à partir des spectres acquis lors de l'étape a) dudit procédé, ces instructions étant aptes à être exécutées par un microprocesseur.

Un troisième objet de l'invention est un dispositif pour l'acquisition de spectres d'un rayonnement transmis par un objet comportant :

une source d'irradiation, apte à émettre un rayonnement électromagnétique ionisant, dit rayonnement incident, vers l'objet;
un détecteur comportant des pixels, chaque pixel étant configuré pour détecter un rayonnement transmis par l'objet vers le détecteur, et à en acquérir un spectre ;
un masque, apte à être interposé entre la source et l'objet, ledit masque comportant des éléments absorbants, aptes à absorber une partie dudit rayonnement incident, et dont une projection sur le détecteur définit des zones d'ombre distantes les unes des autres ;
un processeur, configuré pour recevoir des spectres acquis par chaque pixel, et à mettre en oeuvre les étapes b) à j) du procédé décrit dans cette demande.

L'invention sera mieux comprise par les exemples de réalisation décrits ci-après, lesquels se basent sur les figures décrites ci-après.

## FIGURES

La figure 1A représente un dispositif permettant la mise en oeuvre de l'invention. La figure 1B représente un masque équipant le dispositif, également dessiné sur la figure 1A. La figure 1C représente une région d'analyse et une zone d'ombre d'analyse, formées sur un détecteur.

La figure 2A représente une matrice de réponse du détecteur. Les figures 2B et 2C représentent respectivement une ligne et une colonne de cette matrice de réponse.

La figure 3A représente les principales étapes d'un mode de réalisation de l'invention. La figure 3B représente les principales étapes d'un autre mode de réalisation de l'invention.

La figure 4A représente les étapes permettant d'obtenir une matrice de passage mise en oeuvre dans l'invention. La figure 4B un exemple de matrice de passage. La figure 4C représente une ligne de la matrice de passage.

Les figures 5A et 5B représentent un fantôme pris en compte lors de simulations. Les figures 5C, 5D et 5E représentent des résultats de modélisations simulant une image du fantôme, chaque image étant basée sur des spectres mesurés par les pixels d'un détecteur, ces spectres représentant respectivement le rayonnement total, le rayonnement primaire et l'estimation du rayonnement primaire selon l'invention. La figure 5F représente un profil horizontal obtenu à partir des 5C, 5D et 5E.

Les figures 6A, 6B et 6C représentent des résultats de modélisations simulant une reconstruction tomographie d'une partie centrale du fantôme représenté sur les figures 5A et 5B. Les figures 6A, 6B et 6C sont des images en coupe obtenues respectivement à partir des spectres du rayonnement total, du rayonnement primaire et de l'estimation du rayonnement primaire selon l'invention. La figure 6D représente un profil horizontal obtenu à partir des figures 6A, 6B et 6C.

## EXPOSE DE MODES DE REALISATION PARTICULIERS

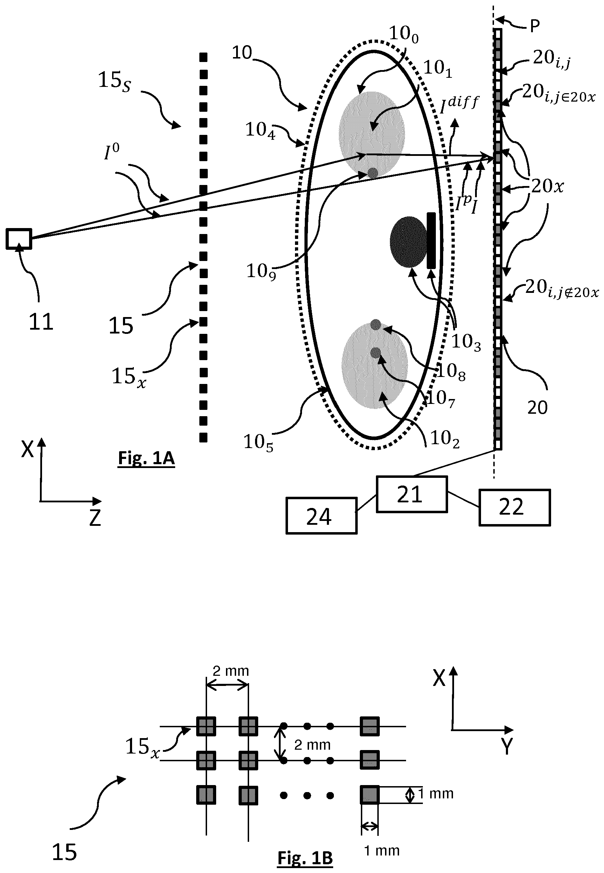

La figure 1A représente un mode de réalisation d'un dispositif 1 mettant en oeuvre un procédé selon l'invention.

Une source d'irradiation 11 émet un rayonnement électromagnétique ionisant $I^0$, dit rayonnement incident, vers un objet 10. L'objet 10 est disposé entre la source d'irradiation 11 et un détecteur de rayonnement 20. Le détecteur de rayonnement est un détecteur comprenant des pixels $20_{i,j}$ agencés selon un plan, dit plan de détection $P$. Les indices $i,j$ désignent les coordonnées, dites coordonnées radiales, de chaque pixel dans le plan de détection. Les pixels peuvent s'étendre selon une ligne, mais en général, ils s'étendent selon une matrice régulière bidimensionnelle.

L'objet 10 peut être un tissu biologique vivant, par exemple une partie du corps d'un animal ou d'un être humain. Le dispositif est alors un dispositif d'imagerie médicale. L'objet peut également être une pièce industrielle ou un bagage, le dispositif étant alors utilisé à des fins de contrôle non destructif ou pour des contrôles liées à la sécurité. Dans cet exemple, l'objet est une partie d'un fantôme représentant un corps humain, comportant une matrice $10_0$, deux poumons $10_1$, $10_2$, une vertèbre $10_3$, des cotes $10_5$ ainsi que des tumeurs, $10_7$, $10_8$ et $10_9$. La matrice $10_0$ est délimitée par un contour $10_4$.

Le terme rayonnement électromagnétique ionisant désigne un rayonnement électromagnétique constitué de photons d'énergie supérieure à 1 keV, et de préférence inférieure à 5 MeV. La plage d'énergie du rayonnement ionisant peut être comprise entre 1 keV et 2 MeV, mais elle s'étend le plus souvent entre 1 keV et 150 keV ou 300 keV. Le rayonnement ionisant peut être un rayonnement X ou $\gamma$. De préférence, la source de rayonnement ionisant est poly-énergétique, le rayonnement incident étant émis selon une plage d'énergie s'étendant généralement selon plusieurs dizaines voire centaines de keV. Il s'agit notamment d'un tube émetteur de rayons X.

Une partie des photons, constituant le rayonnement incident $I^0$, traversent l'objet et atteignent le détecteur 20, sans interagir avec l'objet. Ces photons forment une composante primaire, ou rayonnement primaire $I^p$. D'autres photons constituant le rayonnement incident $I^0$ sont absorbés dans l'objet, par exemple par effet photoélectrique. Enfin, certains photons subissent une interaction de diffusion dans l'échantillon, de type diffusion inélastique Compton ou diffusion élastique Rayleigh. La diffusion, qu'elle soit inélastique ou élastique, engendre un changement de la direction du photon.

Ainsi, l'objet 10 irradié par la source 11 transmet au détecteur 20 un rayonnement $I$, dit rayonnement transmis, comportant :

- une composante directe, ou rayonnement primaire, $I^p$, n'ayant pas interagi avec l'objet, et dont la trajectoire depuis la source est rectiligne ;
- une composante de diffusion $I^{diff}$, ou rayonnement de diffusion, due à une diffusion du rayonnement incident dans l'objet.

Le rayonnement transmis $I$ par l'objet atteint les pixels du détecteur 20, chaque pixel détectant une partie de ce rayonnement. Le rayonnement transmis par l'objet et détecté par un pixel $20_{i,j}$ est noté $I_{i,j}$.

Comme évoqué en relation avec l'art antérieur, le rayonnement de diffusion $I^{diff}$ perturbe l'interprétation des mesures. En effet, contrairement au rayonnement primaire $I^p$, le rayonnement de diffusion se propage depuis l'objet vers le détecteur, selon une direction variable. Ainsi, une partie du rayonnement collecté par chaque pixel $20_{i,j}$ du détecteur ne provient non pas directement de la source de rayonnement 11, mais résulte du phénomène de diffusion. Or, l'interprétation des images est basée sur l'atténuation du rayonnement incident par le détecteur, cette dernière étant obtenue par un ratio, sur une plage d'énergie donnée, entre l'intensité du rayonnement primaire $I^p$ sur l'intensité du rayonnement incident $I^0$. Une bonne interprétation des images suppose la connaissance de l'intensité du rayonnement primaire $I^p$, alors que le rayonnement $I$ transmis par l'objet, et mesuré par le détecteur, comporte une somme dudit rayonnement primaire $I^p$ et du rayonnement diffusé $I^{diff}$.

Chaque pixel $20_{i,j}$ constitue un détecteur de rayonnement, comportant :

- un matériau détecteur, apte à interagir avec les photons du rayonnement $I$ transmis par l'objet, ce matériau étant de type scintillateur ou, de préférence, un matériau semiconducteur compatible avec une utilisation à la température ambiante, de type CdTe, CdZnTe ;
- un circuit électronique, apte à générer un signal dont l'amplitude dépend, et est de préférence proportionnelle, à l'énergie déposée par chaque photon interagissant dans le matériau détecteur ;
- un circuit de spectrométrie, apte à établir un spectre, noté $\mathbf{S}_{i,j}$ en énergie des signaux détectés pendant une période temporelle, dite période d'acquisition.

Ainsi, lorsque les pixels sont disposés régulièrement selon un agencement matriciel, chaque pixel est apte à produire un spectre $\mathbf{S}_{i,j}$ du rayonnement transmis par l'objet. Le détecteur est alors apte à former plusieurs images, chaque image représentant un contenu de chaque spectre dans une plage d'énergie $\Delta E$ déterminée. Typiquement, chaque image comporte l'intégrale ou la valeur moyenne de chaque spectre $\mathbf{S}_{i,j}$ dans ladite bande d'énergie. On parle alors d'imagerie spectrale, puisque le détecteur est à la fois résolu spatialement et spectralement.

Aussi, sous l'effet de l'irradiation par le rayonnement incident $I^0$, l'objet 10 transmet un rayonnement $I$, dit rayonnement transmis, vers un détecteur spectrométrique pixellisé 20, dont chaque pixel $20_{i,j}$ est apte détecter ledit rayonnement transmis *I* et à former un spectre en énergie $S_{i,j}$ du rayonnement $I_{i,j}$ ainsi détecté.

Le terme spectre en énergie correspond à un histogramme de l'amplitude des signaux détectés au cours d'une période d'acquisition du spectre. Une relation entre l'amplitude A et l'énergie E peut être obtenue par une fonction d'étalonnage en énergie *g* telle que E = g(A), selon des principes connus de l'homme du métier. Un spectre d'énergie $S_{i,j}$ est donc un vecteur, dont chaque terme $S_{i,j}$(n) représente une quantité de rayonnement détecté par le pixel 20$_{i,j}$ dans une plage d'énergie $E_n \pm \frac{\partial E}{2}$, avec $\partial E$ étant la largeur spectrale de chaque canal, *n* désignant un numéro de canal. A chaque canal *n* correspond une énergie $E_n$, ou plus exactement une plage d'énergie $E_n \pm \frac{\partial E}{2}$.

Chaque spectre en énergie $S_{i,j}$ peut être considéré comme une somme d'un spectre du rayonnement primaire, noté $S_{i,j}^p$ et d'un spectre du rayonnement de diffusion $S_{i,j}^{diff}$, à un terme de bruit près. Aussi, $S_{i,j} \approx S_{i,j}^p + S_{i,j}^{diff}$ (1). Le signe $\approx$ signifie une égalité à un terme de bruit près, ce bruit pouvant notamment résulter de la source d'irradiation, du détecteur ou d'effets dits d'empilement, se produisant lorsque deux photons incidents sont détectés simultanément. Un objectif de l'invention est de corriger le spectre mesuré par chaque pixel, de façon à réduire la composante de diffusion $S_{i,j}^{diff}$ et d'établir un spectre corrigé $S_{i,j}^*$ tel que $S_{i,j}^* \approx S_{i,j}^p$. Autrement dit, le spectre corrigé $S_{i,j}^*$ correspond à une estimation $\widehat{S}_{i,j}^p$ du spectre du rayonnement primaire atteignant le pixel $20_{i,j} : S_{i,j}^* = \widehat{S}_{i,j}^p$.

Le dispositif comporte également un masque 15, disposé entre la source 11 et le détecteur 20 et dans cet exemple, entre la source 11 et l'objet 10, ce qui constitue la configuration préférée. Ce masque comporte des éléments absorbants 15$_x$ répartis spatialement selon une surface 15$_s$ selon laquelle s'étend le masque. Chaque élement absorbant est apte à atténuer partiellement une partie du rayonnement incident $I^0$ produit par la source d'irradiation. Les éléments absorbants sont répartis discrètement, de telle sorte que l'espace entre deux éléments absorbants adjacents est moins absorbant que lesdits éléments absorbants. Autrement dit, les éléments absorbants définissent une répartition spatiale discrète d'atténuations $att_{15}^x$, $att_{15}^{x'}$ de telle sorte qu'entre deux éléments absorbants adjacents 15$_x$, 15$_{x'}$, l'atténuation $att_{15}^0$ est inférieure à l'atténuation $att_{15}^x$, $att_{15}^{x'}$ associée à chaque élément absorbant.

Le terme atténuation est connu de l'homme du métier. Elle peut être exprimée selon l'expression $att_{15}^x(E) = -ln\left[\frac{I^x(E)}{I^0(E)}\right]$, où $I^0$ (E) désigne une intensité, à une énergie *E,* d'un rayonnement incident $I^0$ à un l'élément absorbant 15$_x$ et $I^x$(E) désigne une intensité, à ladite énergie *E* d'un rayonnement $I^x$ transmis par l'élément absorbant 15$_x$.

L'atténuation peut être exprimée en fonction d'un coefficient d'atténuation linéaire $\mu(E)$, dépendant de l'énergie *E* et du matériau formant le masque, selon l'expression : $att_{15}^x(E) = -\mu(E)\ell_x$, $\ell_x$ désignant l'épaisseur de l'élément atténuant 15$_x$ traversé.

D'une façon générale, l'interposition du masque 15 entre la source 11 et le détecteur 20 ne doit pas modifier significativement le rayonnement de diffusion parvenant au détecteur, par rapport à une configuration sans masque. Aussi, de préférence, chaque élément absorbant présente une atténuation, tel que précédemment défini, comprise entre 0.05 et 1.5, à une des énergies de la plage d'énergie selon laquelle est émis le rayonnement incident $I^0$, ou à l'énergie moyenne de cette plage d'énergie. Ainsi, en négligeant la diffusion, chaque élément absorbant atténue, de préférence, entre 5 % et 80% du rayonnement incident $I^0$ produit par la source et/ou traversant le masque dans l'espace s'étendant entre les éléments absorbants du masque. De préférence, l'atténuation est inférieure à 1 voire inférieur à 0.5 et de préférence inférieur à 0.3. Ainsi, chaque élément absorbant absorbe respectivement moins de 60 %, ou moins de 40%, et de préférence moins de 30% du rayonnement produit par la source, ou du rayonnement passant entre les éléments absorbants du masque. En dessous d'une atténuation égale à 0.05, correspondant à une atténuation de 5% du rayonnement produit par la source, les inventeurs considèrent que l'atténuation n'est pas suffisante. Autrement dit, le masque 15 permet donc établir un contraste d'atténuation, entre les éléments absorbants 15$_x$ et l'espace s'étendant entre lesdits éléments absorbant, ces derniers absorbant entre 5% et 30%, voire 40%, voire davantage du rayonnement traversant ledit espace.

En complément ou alternativement, on peut définir une atténuation globale du masque 15 sous la forme d'un produit d'un facteur de remplissage par le pourcentage du rayonnement incident absorbé par le masque, ce dernier étant déterminé à une énergie de la plage d'énergie du rayonnement incident $I^0$ émis par la source d'irradiation 11, ou à une énergie moyenne de cette plage. Le facteur de remplissage correspond à un ratio entre la surface du masque occupée par l'ensemble des éléments absorbants $15_x$ et la surface totale du masque. L'atténuation globale du masque, ainsi définie, est de préférence supérieure à 0.5% et inférieure à 20%. Ainsi, un masque satisfaisant à cette condition peut avoir un facteur de remplissage égal à 0.2, chaque élément $15_x$ du masque absorbant 10% du rayonnement incident, ce qui donne une atténuation globale du masque, telle que précédemment définie, égale à 0.02 (2%).

Chaque élément absorbant peut avoir une forme quelconque, mais au moins une dimension, dans une direction de la surface $15_S$ selon lequel il s'étend, est inférieure à 5 mm, et de préférence inférieure à 2 mm voire à 1 mm. Dans l'ensemble des modes de réalisation précédemment décrits, le masque s'étend de préférence selon un plan XY parallèle à un plan selon lequel s'étendent les pixels du détecteur.

L'espacement entre deux éléments absorbants adjacents, au niveau du masque, peut être inférieur à 5 mm, et est de préférence compris entre 1 mm et 5 mm. D'une façon générale, l'espacement entre deux éléments absorbants adjacents, après projection sur le détecteur 20, est avantageusement compris entre 1 et 10 cm, et de préférence inférieur à 5 cm ou à 3 cm. Comme décrit par la suite, la projection de chaque élément absorbant $15_x$ sur le détecteur définit une zone d'ombre élémentaire. Chaque zone d'ombre élémentaire s'étend autour d'un point central. Avantageusement, l'espacement entre les points centraux de deux zones d'ombre élémentaires adjacentes est compris entre 1 et 10 cm, et de préférence compris entre 1cm et 5 cm. Par projection, on entend une projection selon la direction de propagation du rayonnement émis par la source.

Un exemple de masque est représenté sur la figure 1B. Dans cet exemple, le masque présente un arrangement régulier des éléments absorbants, ces derniers formant un damier. Chaque élément absorbant $15_x$ est un plot parallélépipédique, dont l'aire, selon la surface $15_S$ selon laquelle s'étend le masque, est de 1 mm * 1 mm, l'espacement entre le centre de chaque élément $15_x$ étant respectivement de 2 mm selon une première direction Y et selon une deuxième direction X perpendiculaire à la première direction. Dans cet exemple, le matériau constituant le masque est de l'Aluminium, et son épaisseur est de 2 mm, ce qui correspond à une valeur d'atténuation, telle que précédemment définie, égale à 0.9 à 100 keV, ce qui conduit à atténuer 10% du rayonnement incident à cette énergie. L'épaisseur s'entend selon une direction perpendiculaire à la surface selon laquelle s'étend le masque. D'une façon générale, le masque est réalisé selon un matériau suffisamment absorbant pour atténuer une portion suffisante du rayonnement primaire. On évite toutefois les matériaux trop denses, de type métaux lourds, par exemple le plomb, susceptibles de produire une diffusion significative du rayonnement incident avant que ce dernier n'atteigne l'objet. Parmi les matériaux préférés composant le masque, on peut citer l'aluminium, le cuivre, le graphite, le bore.

D'autres géométries sont envisageables, en considérant par exemple un espacement non régulier entre les différents éléments absorbants, ou une géométrie non régulière de chaque élément absorbant. Un masque en forme de grille, définissant des mailles, est également possible, les éléments absorbants étant disposés entre chaque maille.

La surface $15_S$ selon laquelle s'étend le masque, entre chaque élément absorbant, est de préférence constituée d'un matériau considéré comme transparent à l'égard des photons, dans la plage d'énergie considérée. Il peut s'agir d'une fine épaisseur d'un plastique, d'un papier ou d'un métal léger, de type aluminium, fer, cuivre, ou un espace laissé libre et occupé par de l'air. Ainsi, entre chaque élément absorbant $15_x$, le coefficient d'atténuation, tel que précédemment défini, est de préférence inférieur à 0.5, voire à 0.2 ou encore 0.1. De préférence, entre chaque élément absorbant, le coefficient d'atténuation est négligeable.

Le nombre d'éléments absorbants est dimensionné de façon à couvrir le champ d'observation du détecteur. Dans l'exemple décrit, les éléments absorbants sont répartis selon une matrice de 34 par 7 éléments, soit un total de 238 éléments.

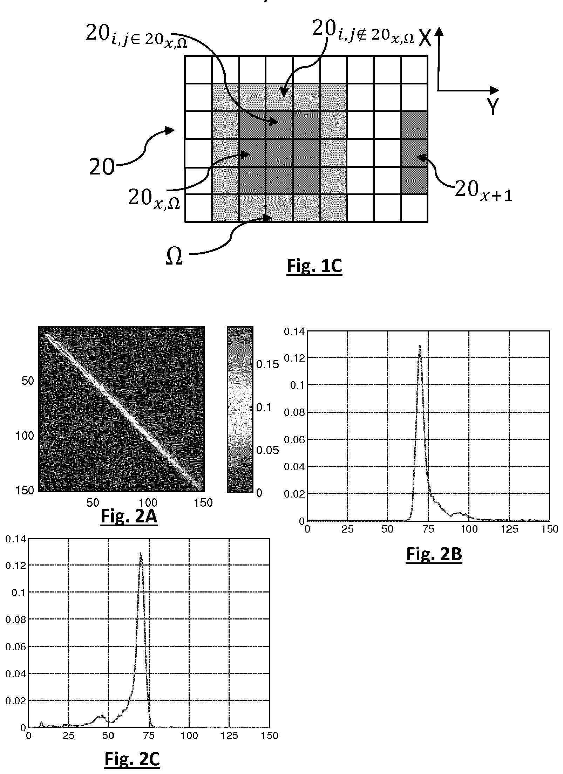

Le masque est interposé entre la source et le détecteur, sa projection, sur le détecteur, selon la direction de propagation du rayonnement incident $I^0$, définit des zones d'ombre $20_x$, chaque zone d'ombre rassemblant les pixels du détecteur $20_{i,j \in 20_x}$ alignés par rapport à chaque élément absorbant $15_x$, selon la direction de propagation du rayonnement atteignant le détecteur. Plus précisément, comme précédemment évoqué, la projection de chaque élément absorbant $15_x$, selon la direction de propagation du rayonnement incident, forme une zone d'ombre $20_x$ sur le détecteur. L'indice $x$ désigne la zone d'ombre considérée. La figure 1C représente une zone d'ombre $20_x$ et une zone d'ombre adjacente $20_{x+1}$. Les pixels $20_{i,j \notin 20_x}$ n'appartenant pas une zone d'ombre $20_x$ reçoivent un rayonnement $I$ non atténué par les éléments absorbants $15_x$, tandis que chaque pixel $20_{i,j \in 20_x}$ appartenant à une zone d'ombre reçoit un rayonnement $I^x$ atténué par un élément absorbant $15_x$, ce dernier étant situé sur une ligne s'étendant entre ledit pixel et la source d'irradiation 11. L'exposant $x$ désigne le fait que le rayonnement $I^x$ atteignant le détecteur au niveau d'une zone d'ombre $20_x$ a été atténué par un élément absorbant $15_x$.

Le dispositif comprend également une unité de calcul, ou processeur 21, par exemple un microprocesseur, est apte à traiter chaque spectre $S_{i,j}$ mesuré par les pixels du détecteur. En particulier, le processeur est un microprocesseur relié à une mémoire programmable 22 dans laquelle est stockée une séquence d'instructions pour effectuer les opérations de traitement de spectres et de calculs décrites dans cette description. Ces instructions peuvent être sauvegardées sur un support d'enregistrement, lisible par le processeur, de type disque dur, CDROM ou autre type de mémoire. Le processeur peut être relié à une unité d'affichage 24, par exemple un écran.

Le détecteur peut être caractérisé par une matrice de réponse $D$ du détecteur, représentant les imperfections de la détection. Cette matrice, de taille $N \times M$, est représentée sur la figure 2A. $N$ est le nombre de canaux $n$ de chaque spectre formé par le détecteur et $M$ est le nombre de canaux selon lesquels le spectre incident au détecteur est discrétisé. $N$ et $M$ sont deux entiers positifs. Dans cet exemple, $N = M = 150$, mais de façon générale, le nombre de canaux du spectre est supérieur à 2, voire supérieur à 10, et peut atteindre plusieurs centaines. Chaque terme $D(u, v)$ de cette matrice représente une probabilité qu'un photon incident au détecteur, d'énergie $v$, soit considéré par le détecteur comme ayant une énergie $u$.

Autrement dit, chaque ligne $D(u, \cdot)$ de la matrice, telle que celle représentée sur la figure 2B, représente une distribution de probabilité de l'énergie $v$ d'un photon atteignant le détecteur lorsqu'un photon d'énergie $u$ est détecté. La figure 2B représente la ligne 70 de la matrice $D$. De façon analogue, chaque colonne $D(\cdot, v)$ de la matrice, telle que celle représentée sur la figure 2C, correspond à une distribution de probabilité de l'énergie détectée $u$ par le détecteur lorsque le photon atteignant le détecteur a une énergie $v$. La figure 2C représente la colonne 70 de la matrice $D$. Plus résolution en énergie du détecteur est fine, plus cette matrice tend vers une matrice diagonale. Dans le cas d'un détecteur parfait, la matrice $D$ est la matrice identité.

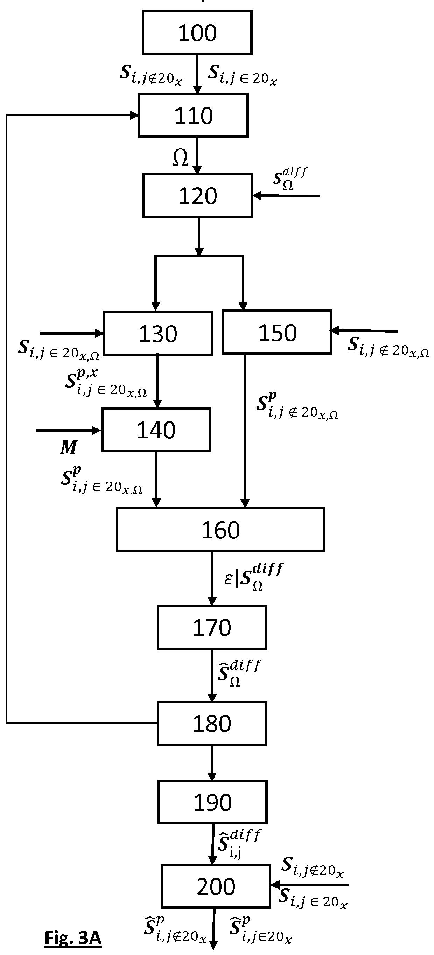

On va à présent décrire les principales étapes d'un procédé selon un premier mode de réalisation de l'invention, en lien avec la figure 3A.

<u>Etape 100</u> : acquisition des spectres transmis par l'objet. Chaque pixel $20_{i,j \in 20_x}$ d'une zone d'ombre $20_x$ acquiert un spectre $\boldsymbol{S}_{i,j \in 20_x}$, transmis par l'objet 10, en subissant une atténuation par un élément absorbant $15_x$. Chaque pixel $20_{i,j \notin 20_x}$ en dehors d'une zone d'ombre $20_x$ acquiert un spectre $S_{i,j \notin 20_x}$, transmis par l'objet 10, sans l'influence d'un élément absorbant $15_x$ du masque 15.

<u>Etape 110</u> : sélection d'une région d'analyse $\Omega$.

Cette étape consiste à sélectionner un pixel, dit pixel sélectionné, et une région d'analyse $\Omega$ s'étendant autour du pixel sélectionné. La région d'analyse est telle qu'elle comporte au moins un pixel, dit pixel intérieur, situé dans une zone d'ombre $20_{i,j \in 20_x}$ et au moins un pixel, dit pixel extérieur $20_{i,j \notin 20_x}$, situé à l'extérieur d'une zone d'ombre. Par ailleurs, la taille de la région d'analyse est suffisamment petite pour qu'on puisse considérer que le rayonnement diffusé par l'objet 10 et atteignant les pixels $20_{i,j \in \Omega}$ de la région d'analyse $\Omega$ puisse être considéré comme étant le même pour tous les pixels de la région d'analyse. Le nombre de pixels $20_{i,j \in \Omega}$ de la région d'analyse $\Omega$ dépend de la taille de ces derniers. La région d'analyse $\Omega$ peut par exemple être carrée, chaque côté étant formé de $a$ pixels, $a$ étant par exemple compris entre 2 et 50. La région d'analyse peut être circulaire, à la forme des pixels près, et s'étendre selon un diamètre de $n$ pixels, $n$ étant par exemple compris entre 2 et 50. La région d'analyse peut être polygonale, par exemple rectangulaire, selon $a$ x $b$ pixels, où $b > a$ et $a \geq 1$.

Un exemple de région d'analyse $\Omega$ est représentée sur la figure 1C. La région d'analyse $\Omega$ comporte :

- des pixels, dits pixels intérieurs $20_{i,j \in 20_{x,\Omega}}$, situés dans une zone d'ombre $20_{x,\Omega}$, dite zone d'ombre d'analyse, située totalement ou partiellement dans la région d'analyse ;
- et des pixels, dits pixels extérieurs $20_{i,j \notin 20_{x,\Omega}}$, situés à l'extérieur de la zone d'ombre d'analyse.

Une zone d'analyse $\Omega$ comporte de préférence une seule zone d'ombre $20_{x,\Omega}$, mais elle peut en comporter plusieurs, en particulier si les zones d'ombres sont faiblement étendues.

<u>Etape 120</u> : Prise en compte d'un spectre du rayonnement diffusé $\boldsymbol{S}_{\Omega}^{diff}$ dans la région d'analyse Cette étape comprend une prise en compte d'un spectre de diffusion $\boldsymbol{S}_{\Omega}^{diff}$ affectant les pixels $20_{i,j \in \Omega}$ de la région d'analyse $\Omega$.

Le spectre de diffusion $\boldsymbol{S}_{\Omega}^{diff}$ pris en compte peut être aléatoire ou se baser sur un a priori, par exemple une condition sur la forme du spectre, ou sur une simulation basée sur un modèle de l'objet examiné, ou sur un spectre de diffusion résultant d'une mise en oeuvre précédente du procédé sur un objet similaire à l'objet 10 examiné, ou celui d'une région d'analyse voisine de la région d'analyse considérée.

Pour les pixels de la région d'analyse $\Omega$ situés à l'intérieur d'une zone d'ombre d'analyse $20_{x,\Omega}$, le spectre $S_{i,j \in 20_{x,\Omega}}$ du rayonnement détecté est formé par l'addition d'un spectre du rayonnement diffusé $\boldsymbol{S}_{\Omega}^{diff}$ affectant la

région d'analyse $\Omega$, et d'un spectre du rayonnement primaire $S^{p,x}_{i,j \in 20_{x,\Omega}}$, dit spectre primaire atténué, transmis par l'objet 10, après absorption (ou atténuation) par l'élément absorbant $15_x$ dont la projection sur le détecteur 20 forme la zone d'ombre $20_x$. L'exposant $p$ désigne le fait que le spectre $S^{p,x}_{i,j \in 20_x}$ est le spectre d'un rayonnement primaire ; l'exposant $x$ désigne l'atténuation par un élément absorbant $15_x$.

Ainsi, pour chaque pixel $20_{i,j \in 20_x}$ appartenant à la zone d'ombre d'analyse $20_{x,\Omega}$ :

$$S_{i,j \in 20_{x,\Omega}} = S^{p,x}_{i,j \in 20_{x,\Omega}} + S^{diff}_{\Omega} \ (2).$$

La région d'analyse $\Omega$ comporte des pixels $20_{i,j \notin 20_{x,\Omega}}$ extérieurs à la zone d'ombre d'analyse $20_{x,\Omega}$. Pour ces pixels, dits extérieurs, le spectre $S_{i,j \notin 20_{x,\Omega}}$ du rayonnement détecté est formé par l'addition du spectre du rayonnement diffusé $S^{diff}_{\Omega}$ affectant la région d'analyse $\Omega$, et d'un spectre du rayonnement primaire $S^p_{i,j \notin 20_{x,\Omega}}$ transmis par l'objet. Ainsi, pour chaque pixel de la zone d'ombre d'analyse $20_{x,\Omega}$ :

$$S_{i,j \notin 20_{x,\Omega}} = S^{p}_{i,j \notin 20_{x,\Omega}} + S^{diff}_{\Omega} \ (3).$$

<u>Etape 130</u> : Calcul du spectre $S^{p,x}_{i,j \in 20_{x,\Omega}}$ du rayonnement primaire atténué détecté par des pixels de la zone d'ombre d'analyse $20_{x,\Omega}$. L'étape 130 est effectuée pour au moins un pixel $20_{i,j \in 20_{x,\Omega}}$ de la zone d'ombre d'analyse $20_{x,\Omega}$ et de préférence pour plusieurs pixels de ladite zone d'ombre d'analyse.

En utilisant l'équation (2), ayant mesuré le spectre $S_{i,j \in 20_{x,\Omega}}$ du rayonnement détecté pour chaque pixel de la zone d'ombre d'analyse lors de l'étape 100, et prenant en compte le spectre du rayonnement diffusé $S^{diff}_{\Omega}$ résultant de l'étape 120, le spectre primaire atténué $S^{p,x}_{i,j \in 20_{x,\Omega}}$ de chaque pixel de la zone d'ombre d'analyse $20_{x,\Omega}$ est obtenu selon l'équation :

$$S^{p,x}_{i,j \in 20_{x,\Omega}} = S_{i,j \in 20_{x,\Omega}} - S^{diff}_{\Omega} \ (4)$$

Cependant, ce spectre est affecté par l'atténuation par le masque, et plus précisément par l'élément absorbant $15_x$, formant la zone d'ombre d'analyse $20_{x,\Omega}$.

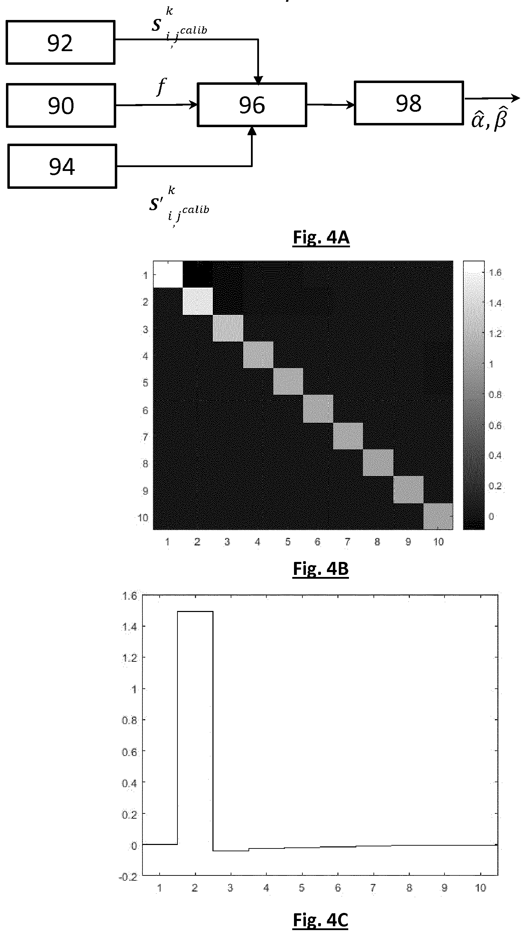

<u>Etape 140</u> : prise en compte de l'atténuation du masque. A partir de chaque spectre primaire atténué $S^{p,x}_{i,j \in 20_{x,\Omega}}$ obtenu lors de l'étape 130, prise en compte de l'atténuation du masque 15 pour estimer le spectre du rayonnement primaire par le pixel de la zone d'ombre d'analyse $20_{x,\Omega}$ en l'absence de masque, ce spectre estimé étant noté $S^{p}_{i,j \in 20_{x,\Omega}}$. Plus précisément, on prend en compte l'atténuation due à l'élément absorbant $15_x$, formant la zone d'ombre d'analyse $20_{x,\Omega}$ pour estimer le spectre primaire que mesurerait le pixel en l'absence du masque. Une telle estimation peut être établie à partir d'un simple calcul de l'atténuation, en fonction de l'énergie, sachant que l'épaisseur et le matériau de l'élément absorbant $15_x$ sont connus. Cependant, de préférence, il est préférable de prendre en compte la réponse du détecteur, précédemment évoquée en lien avec la matrice de réponse **D**. Pour cela, au cours d'une phase de calibrage, on établit une matrice de passage **M** prenant en compte l'atténuation par le masque ainsi que la réponse du détecteur 20. L'établissement de cette matrice de passage est décrit par la suite, en lien avec la figure 4A. L'application de la matrice de passage **M** permet de prendre en compte l'atténuation par l'élément absorbant $15_x$ dont la projection sur le détecteur forme la zone d'ombre d'analyse $20_{x,\Omega}$

En utilisant la matrice de passage ***M,*** le spectre primaire $S^{p}_{i,j \in 20_{x,\Omega}}$' en l'absence d'atténuation, correspondant au pixel de la zone d'ombre d'analyse $20_{x,\Omega}$, est estimé selon l'expression.

$$S^{p}_{i,j \in 20_{x,\Omega}} = M \times S^{p,x}_{i,j \in 20_{x,\Omega}} \quad (5),$$

$\times$ désignant un produit matriciel.

Etape 150 : calcul du spectre du rayonnement primaire détecté par les pixels $20_{i,j \notin 20_{x,\Omega}}$ situés en dehors de la zone d'ombre d'analyse $20_{x,\Omega}$. Pour tout ou partie des pixels $20_{i,j \notin 20_{x,\Omega}}$, le spectre du rayonnement primaire est obtenu, en utilisant l'expression (2), de la façon suivante :

$$S^{p}_{i,j \notin 20_{x,\Omega}} = S_{i,j \notin 20_{x,\Omega}} - S^{diff}_{\Omega} \quad (6)$$

Etape 160: Au cours de l'étape 160, on évalue un écart entre les spectres du rayonnement primaire $S^{p}_{i,j \in \Omega}$ déterminés pour différents pixels de la région d'analyse $\Omega$, les pixels étant respectivement situés à l'intérieur et à l'extérieur de la zone d'ombre d'analyse $20_{x,\Omega}$. Pour les pixels à l'intérieur de la zone d'ombre d'analyse $20_{x,\Omega}$, il s'agit des spectres du rayonnement primaire $S^{p}_{i,j \in 20_{x,\Omega}}$ résultant de l'étape 140. Pour les pixels de la région d'analyse à l'extérieur d'une zone d'ombre, il s'agit des spectres du rayonnement primaire $S^{p}_{i,j \notin 20_{x,\Omega}}$ résultant de l'étape 150. Cette évaluation consiste à définir un indicateur d'écart $\varepsilon | S^{diff}_{\Omega}$ représentant une variation spatiale entre les différents spectres du rayonnement primaire déterminés dans les pixels de la région d'analyse. L'écart $\varepsilon | S^{diff}_{\Omega}$ dépend du spectre de diffusion $S^{diff}_{\Omega}$ pris en compte lors de l'étape 120, car les spectres du rayonnement primaire utilisés pour l'établir sont calculés en prenant en compte le spectre de diffusion $S^{diff}_{\Omega}$. L'indicateur de variation peut notamment être déterminé en fonction d'un gradient spatial $\nabla S^{p}_{i,j}$ du spectre primaire calculé en chaque pixel de la région d'analyse. L'indicateur d'écart peut par exemple être une grandeur scalaire formée par une somme de la valeur absolue de chaque gradient :

$$\varepsilon | S^{diff}_{\Omega} = \sum_{i,j \in \Omega} \left| \nabla S^{p}_{i,j} \right| \quad (7)$$

où $\nabla S^{p}_{i,j}$ représente un gradient spatial de chaque spectre primaire $S^{p}_{i,j}$ résultant de l'étape 140 $(S^{p}_{i,j \in 20_{x,\Omega}})$ ou 150 $(S^{p}_{i,j \notin 20_{x,\Omega}})$. Dans ce cas, plus l'écart $\varepsilon | S^{diff}_{\Omega}$ est faible, plus la variation spatiale des spectres primaires est faible dans la région d'analyse.

Autrement dit, l'indicateur d'écart $\varepsilon | S^{diff}_{\Omega}$ quantifie une variation spatiale des spectres primaires, résultant des étapes 140 et 150, respectivement associés à des pixels à l'intérieur ou à l'extérieur de la zone d'analyse d'intérêt.

Par exemple, la région d'analyse $\Omega$ est un carré centré sur une zone d'ombre d'analyse. L'étape 150 peut être effectuée sur quatre pixels $20_{i,j \notin 20_{x,\Omega}}$ formant respectivement les quatre coins de la région d'analyse. Les étapes 130 et 140 peut être effectuée sur un pixel $20_{i,j \in 20_{x,\Omega}}$ situé au centre de la zone d'ombre d'analyse $20_{x,\Omega}$ L'indicateur d'écart peut alors être défini selon l'expression :

$$\varepsilon | S_\Omega^{diff} = \left\| \nabla S_{20_{i,j \in 20_{x,\Omega}}}^p \right\| \ (7.1)$$

avec :

$$\nabla S_{20_{i,j \in 20_{x,\Omega}}}^p = S_{i,j \in 20_{x,\Omega}}^p - \frac{1}{4} \sum S_{i,j \notin 20_{x,\Omega}}^p \ (7.2)$$

Selon cet exemple, on détermine une moyenne des quatre spectres de diffusion primaires déterminés sur chaque pixel $20_{i,j \notin 20_{x,\Omega}}$, cette moyenne étant soustraite, terme à terme, du spectre $S_{i,j \in 20_{x,\Omega}}^p$ du rayonnement primaire estimé, lors de l'étape 140, pour le pixel $20_{i,j \, 20_{x,\Omega}}$ de façon à former un vecteur de gradient $\nabla S_{20_{i,j \in 20_{x,\Omega}}}^p$, dont la norme $\left\| \nabla S_{20_{i,j \in 20_{x,\Omega}}}^p \right\|$ forme l'indicateur d'écart $\varepsilon | S_\Omega^{diff}$.

Selon un autre exemple, on détermine une valeur moyenne $\overline{S_{i,j \notin 20_{x,\Omega}}^p}$ de différents spectres $S_{i,j \notin 20_{x,\Omega}}^p$ obtenus, lors de l'étape 150, au niveau de pixels $20_{i,j \notin 20_{x,\Omega}}$ situés à l'extérieur de la zone d'ombre d'intérêt $20_{x,\Omega}$ Pour chaque spectre $S_{i,j \in 20_{x,\Omega}}^p$ déterminé lors de l'étape 140 au niveau d'un pixel de la zone d'ombre d'intérêt $20_{x,\Omega}$, le vecteur de gradient est tel que

$$\nabla S_{20_{i,j \in 20_{x,\Omega}}}^p = S_{i,j \in 20_{x,\Omega}}^p - \overline{S_{i,j \notin 20_{x,\Omega}}^p} \ (7.3).$$

L'indicateur d'écart correspondant à la région d'analyse $\Omega$ considérée est alors tel que

$$\varepsilon | S_\Omega^{diff} = \sum_{i,j \in 20_{x,\Omega}} \left| \nabla S_{20_{i,j \in 20_{x,\Omega}}}^p \right| \ (7.4)$$

D'une façon générale, au delà de ces exemples non limitatifs, on comprend que le vecteur de gradient spatial $\nabla S_{i,j}^p$ associé à un pixel $20_{i,j}$ résulte d'une comparaison, notamment sous la forme d'une soustraction ou de ratio, entre de spectres de diffusion primaire, ou de leur valeurs moyennes, respectivement déterminés, au cours des étapes 140 et 150, au niveau de pixels $20_{i,j \in 20_{x,\Omega}}$, $20_{i,j \notin 20_{x,\Omega}}$ situés à l'intérieur ou à l'extérieur de la zone d'ombre d'analyse $20_{x,\Omega}$.

<u>Etape 170</u> : Estimation du spectre du rayonnement de diffusion $\widehat{S}_\Omega^{diff}$ relatif à la région d'analyse $\Omega$ considérée minimisant l'indicateur d'écart $\varepsilon | S_\Omega^{diff}$.

Cette étape consiste à définir le spectre du rayonnement de diffusion $\widehat{S}_\Omega^{diff}$, pris en compte lors de l'étape 120, minimisant l'écart entre les différents spectres du rayonnement primaire déterminés pour la région d'analyse $\Omega$. Autrement dit,

$$\widehat{S}_\Omega^{diff} = \underset{S_\Omega^{diff}}{\mathrm{argmin}} \left( \varepsilon | S_\Omega^{diff} \right) \ (8)$$

Lorsque l'indicateur de gradient est celui décrit en lien avec l'équation (7),

$$\widehat{S}^{diff}_{\Omega} = \underset{S^{diff}_{\Omega}}{\mathrm{argmin}} \left( \sum_{i,j \in \Omega} \left| \nabla S^{p}_{i,j} \right| \right) (9)$$

Les étapes 120 à 170 forment un processus de minimisation visant à déterminer le spectre de diffusion $\widehat{S}^{diff}_{\Omega}$ minimisant l'indicateur d'écart calculé lors de l'étape 160. Au cours du processus de minimisation, les étapes 120 à 160 sont répétées, en considérant successivement différents spectres de diffusion $S^{diff}_{\Omega}$, de façon à obtenir le spectre de diffusion minimisant l'écart $\widehat{S}^{diff}_{\Omega} = \underset{S^{diff}_{\Omega}}{\mathrm{argmin}}\left(\varepsilon | S^{diff}_{\Omega}\right)$.

Selon un mode de réalisation, les étapes 120 à 170 sont réalisées indépendamment pour différentes bandes d'énergie. Le spectre de diffusion $\widehat{S}^{diff}_{\Omega}$ est alors estimé dans chaque bande d'énergie, indépendamment les unes des autres. Selon un autre mode de réalisation, les étapes 120 à 170 en considérant successivement différentes bandes d'énergie selon lesquelles le spectre $\widehat{S}^{diff}_{\Omega}$ est défini. Selon ce mode de réalisation, le processus de minimisation est effectué successivement en commençant par une bande d'énergie étroite, et adressant les énergies maximales que l'on souhaite adresser. Le processus de minimisation est répété en élargissant progressivement la bande d'énergie considérée vers les faibles énergies. Le spectre de diffusion $\widehat{S}^{diff}_{\Omega}$ est alors estimé progressivement, selon une bande d'énergie s'élargissant peu à peu au cours de chaque réalisation du processus de minimisation.

Etape 180 : réitération en modifiant la région d'analyse $\Omega$ : les étapes 110 à 170 sont de préférence réalisées pour différentes régions d'analyse $\Omega$. Les différentes zones d'analyse peuvent être adjacentes les unes des autres, ou se chevaucher partiellement. Lorsque les différentes zones d'analyse sont adjacentes les unes des autres, sans se chevaucher, à chaque pixel de la zone d'analyse correspond le spectre de diffusion $\widehat{S}^{diff}_{\Omega}$ estimé pour ladite zone d'analyse :

$$\widehat{S}^{diff}_{i,j \in \Omega} = \widehat{S}^{diff}_{\Omega} (10).$$

Selon une variante, les étapes 110 à 170 sont effectuées pixels par pixel, à chaque pixel $20_{i,j}$ correspondant une région d'analyse $\Omega_{i,j}$. Chaque région d'analyse $\Omega_{i,j}$ peut notamment former un noyau, dont le centre correspond au pixel, le noyau étant déplacé d'un pixel à un autre entre deux itérations successives. Dans ce cas, lorsque les pixels sont situés en bordure du détecteur, le noyau est tronqué selon les limites du détecteur 20. On obtient alors autant de spectres de diffusion estimés que de pixels. A chaque pixel $20_{i,j}$ du détecteur correspond alors un spectre de diffusion

$$\widehat{S}^{diff}_{\Omega_{i,j}} : \widehat{S}^{diff}_{i,j} = \widehat{S}^{diff}_{\Omega_{i,j}} (11).$$

Une telle variante peut être mise en oeuvre sur certains pixels seulement du détecteur de façon à optimiser le temps de calcul. Le détecteur peut faire l'objet d'un maillage, par exemple un maillage régulier, de façon à sélectionner les pixels $20_{i,j^*}$ pour lesquelles une région d'analyse $\Omega_{i,j^*}$ est définie, la région d'analyse faisant l'objet des étapes 110 à 170. A chaque pixel $20_{i,j^*}$ sélectionné par le maillage correspond un spectre de diffusion $\widehat{S}^{diff}_{\Omega_{i,j^*}}$. Dans ce cas, à chaque pixel sélectionné $20_{i,j^*}$ est attribué un voisinage, et le spectre de diffusion $\widehat{S}^{diff}_{\Omega_{i,j^*}}$ correspondant au pixel du maillage $\widehat{S}^{diff}_{\Omega_{i,j^*}}$ est attribué aux pixels formant le voisinage.

Selon une possibilité, les étapes décrites ci-avant sont mises en oeuvre pour certaines régions d'analyse $\Omega$, et à chaque région d'analyse $\Omega$ est attribué un spectre de diffusion $\widehat{S}_{\Omega}^{diff}$. Une interpolation spatiale peut être effectuée, de façon à attribuer, à chaque pixel $20_{i,j}$ du détecteur 20, un spectre de diffusion $\widehat{S}_{i,j}^{diff}$.

A l'issue de l'étape 180, à chaque pixel $20_{i,j}$ du détecteur 20 est associé un spectre de diffusion $\widehat{S}_{i,j}^{diff}$.

Etape 190 : Lissage

Afin d'éviter des discontinuités trop importantes entre les spectres de diffusion $\widehat{S}_{i,j}^{diff}$ associés à des pixels $20_{i,j}$, en particulier des pixels adjacents, résultant de l'étape 180, un lissage entre ces différents spectres de diffusion peut être prévu.

Etape 200 : Correction

Cette étape permet une correction du spectre du rayonnement $S_{i,j}$ acquis par chaque pixel $20_{i,j}$ du détecteur 20 en fonction du spectre de diffusion $\widehat{S}_{i,j}^{diff}$ associé à chacun d'entre eux, de façon à obtenir un spectre corrigé $S^*_{i,j}$, ce dernier étant une estimation du spectre primaire $\widehat{S}_{i,j}^{p}$. Pour les pixels $20_{i,j \notin 20_x}$ situés en dehors d'une zone d'ombre, le spectre corrigé $S^*_{i,j \notin 20_x}$ correspond à l'estimation du spectre de rayonnement primaire selon l'expression :

$$S^*_{i,j \notin 20_x} = \widehat{S}_{i,j \notin 20_x}^{p} = S_{i,j \notin 20_x} - \widehat{S}_{i,j \notin 20_x}^{diff} \ (12).$$

Pour les pixels $20_{i,j \in 20_x}$ situés dans une zone d'ombre, le spectre corrigé $S^*_{i,j \in 20_x}$ correspond à l'estimation du spectre de rayonnement primaire selon l'expression :

$$\widehat{S}_{i,j \in 20_x}^{p} = M \times \left( S_{i,j \in 20_x} - \widehat{S}_{i,j \in 20_x}^{diff} \right) (13)$$

$$\text{soit } \widehat{S}_{i,j \in 20_x}^{p} = M \times \left( S_{i,j \in 20_x}^{p,x} \right) (13')$$

$$\text{avec } S_{i,j \in 20_x}^{p,x} = S_{i,j \in 20_x}^{p} - \widehat{S}_{i,j \in 20_x}^{diff} (13'')$$

$S_{i,j \notin 20_x}$ et $S_{i,j \in 20_x}$ désignent respectivement des spectres respectivement mesurés par des pixels situés en dehors et dans une zone d'ombre $20_x$.

On dispose ainsi d'une estimation du spectre primaire pour chaque pixel du détecteur.

Entre les étapes 100 et 110, chaque spectre mesuré peut faire l'objet d'un regroupement spectral en plages d'énergies : certains canaux sont regroupés, de façon à réduire le nombre de canaux.

Les spectres primaires obtenus par le procédé peuvent être utilisés pour former des images spectrales, une image spectrale correspondant au contenu des spectres primaires dans une plage d'énergie déterminée.

Dans le cas d'une reconstruction tomographique, le procédé est mis en oeuvre dans différentes configurations *C,* à chaque configuration correspondant une position relative du détecteur 20 et/ou de la source 11 par rapport à l'objet 10. La reconstruction tomographique est réalisée sur la base des spectres primaires obtenus par le procédé ou des images spectrales formées à partir des spectres primaires.

Etablissement de la matrice de passage.

Comme préalablement décrit en lien avec l'étape 140, la prise en compte de l'atténuation par un élément absorbant $15_x$ du masque 15 peut être effectuée par une matrice de passage **M.** Selon une première approximation, basée sur une hypothèse d'un détecteur parfait, selon lequel la matrice de réponse **D** correspond à la matrice identité, la matrice de passage peut être déterminée à partir d'une matrice dite matrice d'atténuation **C,** de préférence diagonale, dont chaque terme correspond à une atténuation dans une plage d'énergie donnée. Plus précisément, chaque terme

$$C(p,p) = e^{-\mu_x \ell_x} \ (14)$$

- $\mu_x$ est le coefficient d'atténuation linéaire, dans la bande d'énerge $\Delta E_p$ associée a la ligne $p$ de la matrice d'atténuation **C;**
- $\ell_x$ est l'épaisseur de l'élément absorbant $15_x$ dont la projection sur le masque 15 forme la zone d'ombre $20_x$ considérée.

Ainsi, sans prise en compte de la réponse du détecteur, ou en considérant un détecteur parfait, **M = C⁻¹.**

Cependant, une approche plus rigoureuse consiste à prendre en compte la réponse du détecteur, par le biais de la matrice de réponse **D.** Selon une telle approche, la matrice de passage **M** peut être exprimée comme suit :

$$M = D \times C^{-1} \times D^{-1} \ (15)$$

Cela dit, la matrice de réponse **D** n'est pas généralement pas inversible.

Les inventeurs ont mis en place une détermination expérimentale de la matrice **M** selon les étapes décrites ci-dessous. On a considéré que la matrice **M** est triangulaire et que chaque ligne $p$ de la matrice **M** est telle que :

$$M\,(p,.) = C(p,p)^{-1} f \ (16)$$

où f est une fonction dite de déformation, telle que :

-

$$f(p, q, \alpha(p), \beta(p)) = (1 - \beta)\delta(q) - \beta e^{-\alpha q} \ (17)$$

lorsque $p \geq q$ et

$$f(p, q, \alpha(p), \beta(p)) = 0 \text{ lorsque } p < q \ (18)\ ;$$

- $\alpha(p)$ et $\beta(p)$ sont des scalaires déterminés pour chaque ligne $p,$ chaque ligne représentant une plage d'énergie ;
- $q$ désigne une colonne de la matrice **M,** chaque colonne étant associée à une plage d'énergie ;
- la fonction $\delta(q)$ est une fonction de Dirac, avec $\delta(q) = 1$ si $p = q$ et 0 sinon ;

La fonction de déformation $f$ permet d'appliquer une déformation à l'atténuation du masque, représentée par les termes $C(p, p)^{-1}$, en prenant en compte les imperfections de la détection. Pour déterminer la matrice de passage **M,** connaissant la matrice d'atténuation **C** et l'expression de la fonction de déformation $f,$ il faut déterminer les paramètres $\alpha$ et $\beta$ de la fonction $f$.

La matrice de passage **M** peut être déterminée sur la base de simulations numériques, mais elle peut également être déterminée expérimentalement, en effectuant des acquisitions respectivement avec et sans masque 15 interposé entre le détecteur 20 et la source d'irradiation 10. Chaque acquisition est réalisée en disposant un objet de calibration $10_k$ entre la source 11 et le détecteur 20. Différents objets de calibration $10_k$ sont successivement utilisés, l'indice k désignant chaque objet de calibration, avec $1 < k < K$, K désignant le nombre d'objets de calibration utilisés.

Par exemple, chaque objet de calibration $10_k$ est constitué d'un ou plusieurs matériaux de calibration d'épaisseurs connues. Dans cet exemple, on a utilisé deux matériaux de calibration, en l'occurrence l'eau et l'aluminium. Différents objets de calibration ont été constitués, l'épaisseur d'eau étant variable, entre 1 et 20 cm, par pas de 0.5 cm, et l'épaisseur d'aluminium étant variable, entre 0.1 et 2 cm, par pas de 0.1 cm.

Les étapes de la calibration, représentées sur la figure 4A, sont les suivantes :

Etape 90 : détermination de la forme générale de la fonction de déformation *f*. Cela revient à exprimer analytiquement la fonction, en fonction de paramètres selon les équations (17) et (18);

Etape 92 : placement d'un objet de calibration $10_k$ entre la source 11 et le détecteur 20, un masque 15 étant disposé entre la source et le détecteur, puis acquisition d'un premier spectre de calibration $S_{i,j\,calib}^{k}$ par un pixel $20_{ij}^{calib}$, dit pixel de calibration, présent à l'intérieur d'une zone d'ombre $20_x$ formée par la projection d'un élément absorbant $15_x$ du masque. Le premier spectre de calibration prend en compte l'atténuation dudit élément absorbant.

Etape 94 : retrait du masque et acquisition d'un deuxième spectre de calibration $S'_{i,j\,calib}^{k}$ par le pixel de calibration $20_{i,j calib}$. Le deuxième spectre de calibration ne subit pas l'influence du masque.

Etape 96 : arrêt de la calibration ou réitération des étapes 92 à 96 en utilisant un autre objet de calibration.

Etape 98 : détermination des paramètres $\hat{\alpha}, \hat{\beta}$ de la fonction de déformation *f* à l'aide des spectres $S'_{i,j\,calib}^{k}$ et $S_{i,j\,calib}^{k}$ mesurés lors de chaque étape 92 et 94. Ces paramètres sont ceux minimisant un écart moyen ou cumulé entre respectivement :

- un spectre mesuré sans atténuation, $S'_{i,j\,calib}^{k}$ correspondant à un objet de calibration $10_k$;

- le produit matriciel de la matrice de passage *M* par le spectre $S_{i,j\,calib}^{k}$ mesuré avec l'atténuation par le masque 15, à l'aide dudit objet de calibration.

$\hat{\alpha}(p)$ et $\hat{\beta}(p)$ peuvent être obtenus selon l'expression suivante :

$$\hat{\alpha}(p), \hat{\beta}(p) = \operatorname*{argmin}_{\alpha,\,\beta} \sum_{k=1}^{K} \left| S'_{i,j\,calib}^{k}(p) - \sum_{q} M(p,q) \times S_{i,j\,calib}^{k}(q) \right| \quad (19)$$

Ainsi, à chaque plage d'énergie considérée, représentée par l'indice *p*, les coefficients $\hat{\alpha}(p)$ et $\hat{\beta}(p)$ sont ceux minimisant un écart, à cette énergie, entre :

- des spectres $S'_{i,j\,calib}^{k}$ transmis par chaque objet de calibration en l'absence d'un masque 15,

- et l'estimation des spectres $S'_{i,j\,calib}^{k}$ à l'aide de spectres $S_{i,j\,calib}^{k}$ mesurés en présence du masque 15, chaque estimation étant formée par un produit entre la matrice de déformation *M* et le spectre mesuré en présence du masque.

A l'aide des spectres de calibration mesurés, avec et sans masque, on estime $\hat{\alpha}(p)$ et $\hat{\beta}(p)$ à l'aide de l'équation (19).

La figure 4B représente une matrice de passage *M,* chaque ligne *p* et chaque colonne *q* représentant une bande d'énergie de largeur 10 keV. La figure 4C représente les valeurs de la matrice selon la deuxième ligne (*p* = 2).

Selon une variante, lors de la détermination de la matrice de passage, un seuil est appliqué à la matrice d'atténuation inversée $C^{-1}$, pour éviter que certains termes tendent vers l'infini, et cela concerne en particulier les valeurs d'atténuation aux faibles énergies.

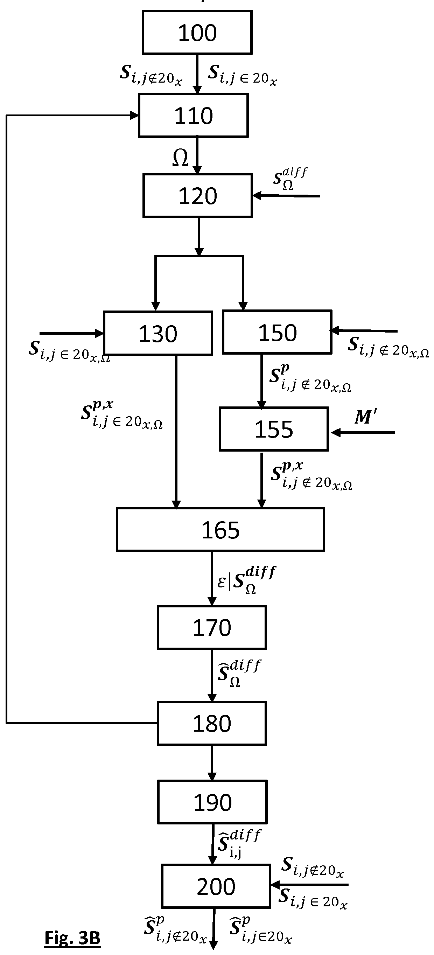

Selon un autre mode de réalisation, représenté sur la figure 3B, les étapes 100 à 130, et 150 sont identiques à celles précédemment décrites. Le procédé comporte ensuite les étapes suivantes : Etape 155 : à partir de chaque spectre primaire obtenu lors de l'étape 150, pour les pixels situés en dehors de la zone d'ombre d'analyse $20_{x,\Omega}$, prise en compte de l'atténuation du masque 15 pour estimer le spectre du rayonnement primaire détecté par chaque pixel en présence d'un élément absorbant $15_x$ entre la source et le détecteur. De même que lors de l'étape 140 préalablement décrite, on prend en compte une matrice de passage *M'* non pas pour estimer, à partir d'un spectre primaire atténué $S_{i,j\in 20_{x,\Omega}'}^{p,x}$ un spectre primaire non atténué $S_{i,j\in 20_{x,\Omega}'}^{p}$ mais pour estimer, à partir d'un spectre primaire non atténué

$S^p_{i,j \notin 20_{x,\Omega}}$, car mesuré par un pixel en dehors de la zone d'ombre d'analyse $20_{x,\Omega}$, un spectre primaire atténué

$S^{p,x}_{i,j \notin 20_{x,\Omega}}$ par l'élément absorbant $15_x$ formant la zone d'ombre d'analyse $20_{x,\Omega}$. On prend en compte l'atténuation due à l'élément absorbant, formant la zone d'ombre d'analyse $20_{x,\Omega}$, pour estimer le spectre primaire $S^{p,x}_{i,j \notin 20_{x,\Omega}}$ que mesurerait chaque pixel à l'extérieur de la zone d'ombre d'analyse s'il était atténué par le masque formant ladite zone d'ombre d'analyse. De façon analogue à l'étape 140, le spectre primaire atténué peut être estimé à l'aide d'une matrice de passage $M'$, de telle sorte que $S^{p,x}_{i,j \notin 20_{x,\Omega}} = M' \times S^p_{i,j \notin 20_{x,\Omega}}$.

La matrice de passage $M'$ peut être déterminée en prenant en compte l'atténuation du masque. IL peut alors s'agir simplement d'une matrice diagonale

$$M' = \mathcal{I} - C^{-1},$$

$\mathcal{I}$ étant la matrice identité et $C$ étant la matrice d'atténuation précédemment déterminée. De préférence, la matrice de passage $M'$ prend en compte la réponse du détecteur et est déterminée de façon analogue à la matrice $M$, comme décrit en lien avec les étapes 90 à 98, avec :

$$M' = (\mathcal{I} - C^{-1})g \quad (17.1),$$

$g$ étant une fonction de déformation pouvant être déterminée expérimentalement ou numériquement de façon analogue à la fonction de déformation $f$ décrite en lien avec l'équation 16.

<u>Etape 165</u> : Au cours de l'étape 165, on évalue une variation spatiale entre les spectres du rayonnement primaire $S^p_{i,j \in \Omega}$ déterminés pour les différents pixels de la région d'analyse. Pour les pixels de zone d'ombre d'analyse $20_{x,\Omega}$, il s'agit des spectres du rayonnement primaire $S^{p,x}_{i,j \in 20_{x,\Omega}}$ résultant de l'étape 130. Pour les pixels en dehors de la zone d'ombre d'analyse $20_{x,\Omega}$, il s'agit des spectres du rayonnement primaire $S^{p,x}_{i,j \notin 20_{x,\Omega}}$ résultant de l'étape 155.

Cette évaluation consiste à définir un indicateur d'écart $\varepsilon|S^{diff}_\Omega$ représentant une variation spatiale entre les différents spectres du rayonnement primaire déterminés dans les pixels de la région d'analyse. De même que lors de l'étape 160, l'indicateur $\varepsilon|S^{diff}_\Omega$ dépend du spectre de diffusion $S^{diff}_\Omega$ pris en compte lors de l'étape 120. L'indicateur d'écart peut notamment être déterminé en fonction d'un gradient spatial $\nabla S^p_{i,j}$ du spectre primaire calculé en chaque pixel de la région d'analyse. L'indicateur peut par exemple être formé par une somme de la valeur absolue de chaque gradient :

$$\varepsilon|S^{diff}_\Omega = \sum_{i,j \in \Omega}\left|\nabla S^p_{i,j}\right| \quad (7)$$

Dans ce cas, plus l'indicateur de variation $\varepsilon|S^{diff}_\Omega$ est faible, plus la variation spatiale des spectres primaires est faible dans la région d'analyse.

De même qu'indiqué en lien avec l'étape 160, l'indicateur $\varepsilon|S^{diff}_\Omega$ est obtenu par comparaisons entre des spectres primaires estimés pour des pixels situés respectivement à l'intérieur et à l'extérieur de la zone d'ombre d'analyse.

Selon ce mode de réalisation, le procédé comporte les étapes 170 à 200 telles que précédemment décrites.

Exemples

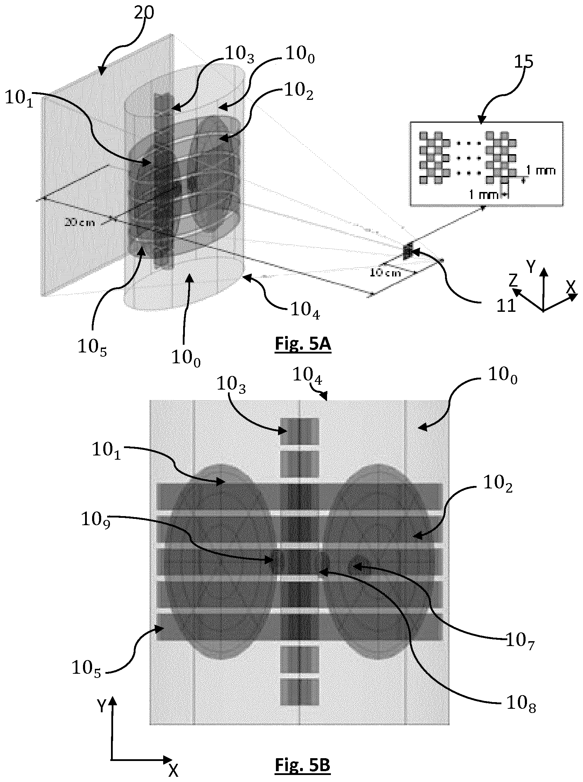

On va à présent décrire des exemples de réalisations de l'invention, basés sur des simulations réalisées par code de calcul, selon une application en radiographie et une application en tomographie. Les simulations mettent en oeuvre le dispositif représenté sur la figure 1A. L'objet 10, représenté sur les figures 1A, 5A et 5B est un fantôme simulant le torse d'un être humain. Il comporte une matrice $10_0$, dont la section, dans un plan parallèle au plan XZ du faisceau d'irradiation, est ovale, les plus grandes dimensions selon les axes Z et X étant respectivement de 20 cm et de 40 cm. Cette matrice $10_0$ représente le corps et est constituée d'eau. Un premier élément $10_1$ et un deuxième élément $10_2$ disposés à l'intérieur de la matrice $10_0$, représentent les poumons. Leur section, dans le plan XZ, est ellipsoïdale, les plus grandes dimensions selon les axes Z et X étant respectivement de 11 cm et de 15 cm. Ces éléments sont constitués d'air. Un troisième élément $10_3$ représente une vertèbre. Sa section, dans le plan XZ, est combine une forme de disque de diamètre 3 cm, et une forme rectangulaire, représente une vertèbre. Le matériau le constituant est un os, modélisé selon une base de données de l'ICRU (International Commission on Radiation Units and Measurements). Enfin, au voisinage de la périphérie de la matrice s'étend un os annulaire $10_5$ de section ellipsoïdale, représentant les cotes. Son épaisseur est de 1 cm. Des inclusions sensiblement sphériques $10_7$, $10_8$ et $10_9$ de diamètre 2 cm sont disposées au niveau des premier et deuxième éléments, représentant les poumons. Ces inclusions sont constituées de PMMA (Polyméthacrylate de méthyle) et représentent des tumeurs cancéreuses. La source de rayonnement 11 est positionnée à 10 cm du masque, et l'objet est positionné à 20 cm du détecteur. La matrice est délimitée par une enveloppe annulaire $10_4$ de section ellipsoïdale.

La source d'irradiation 11 modélisée est un tube à rayons X avec une anode de tungstène, soumis à une tension de 110 kV. Le détecteur 20 comprend 512 (selon l'axe X) * 512 pixels (selon l'axe Y), chaque pixel comprenant une épaisseur de CdTe de 5 mm. La surface de chaque pixel, dans le plan XY selon lequel s'étend le détecteur, est de 1mm * 1mm. Le détecteur est résolu en énergie, et chaque pixel permet d'obtenir des spectres selon des canaux d'énergie de 10 keV. Le masque utilisé est celui représenté sur la figure 1B. L'épaisseur de chaque élément absorbant 15x est de 5 mm. La taille du masque est de 51 mm * 51 mm dans le plan XY.

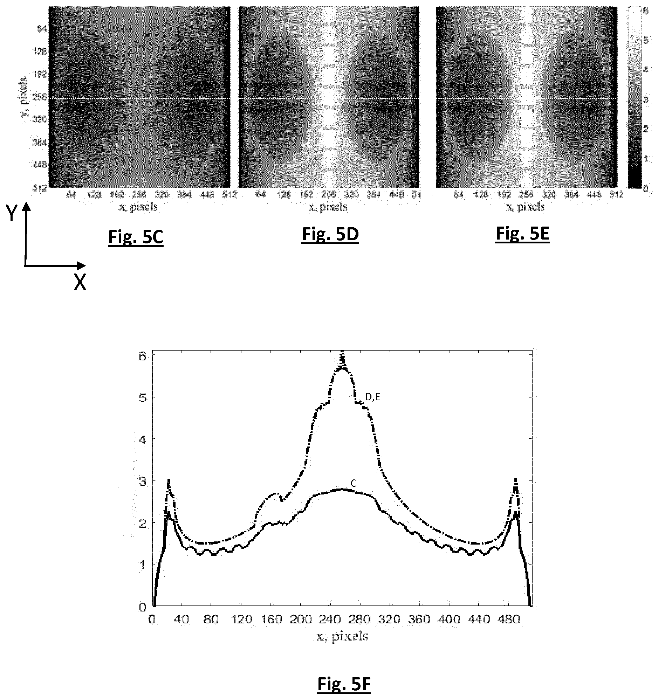

Les figures 5C, 5D et 5E représentent respectivement le rayonnement total sans le masque, le rayonnement primaire et le rayonnement primaire estimé en mettant en oeuvre l'invention, selon le procédé représenté sur la figure 3A, sur l'ensemble des pixels du détecteur. Soit $S_{i,j}^0$ un spectre mesuré par un pixel $20_{i,j}$ lorsque le détecteur est directement exposé à la source, sans masque ni objet entre la source et le détecteur. Les grandeurs représentées, en niveaux de gris, sur chaque pixel $20_{i,j}$ des figures 5C, 5D et 5E sont respectivement :

$$A_{i,j} = -log\left(\frac{\sum_n S_{i,j}(n)}{\sum_n S_{i,j}^0(n)}\right) (21),$$

$n$ désignant un canal du spectre ;

$$A_{i,j} = -log\left(\frac{\sum_n S_{i,j}^p(n)}{\sum_n S_{i,j}^0(n)}\right) (22),$$

$S_{i,j}^p$ désignant le spectre primaire modélisé ;

$$A_{i,j} = -log\left(\frac{\sum_n \widehat{S}_{i,j}^p(n)}{\sum_n S_{i,j}^0(n)}\right) (22),$$

$\widehat{S}_{i,j}^p$ désignant le spectre primaire estimé en mettant en oeuvre les étapes 100 à 200 préalablement décrites, à partir du spectre $S_{i,j}$

Dans les équations (21), (22) et (23), $S_{i,j}$ et $S_{i,j}^0$ représentent respectivement les modélisations du spectre détecté par un pixel $20_{i,j}$ en présence et en l'absence du fantôme.

Chaque grandeur $A_{i,j}$ est représentative d'une atténuation globale dans l'objet dans la plage d'énergie 10 keV - 110 keV, le terme "globale" désignant le fait qu'elle est déterminée pour l'ensemble des canaux d'énergie du spectre. Cela permet une représentation de chaque spectre par un scalaire, ce qui facilite les illustrations. Les figures 5C, 5D et 5E montrent que :

- l'estimation du spectre du rayonnement primaire $\hat{S}_{i,j}^{p}$, mettant en oeuvre l'invention (figure 5E) est cohérente avec le spectre du rayonnement primaire modélisé $S_{i,j}^{p}$ (figure 5B) ;

- les spectres du rayonnement primaire sont plus contrastés (figures 5D et 5E) que le spectre du rayonnement total (figure 5C). La correction effectuée par l'invention, permettant d'obtenir le spectre du rayonnement primaire, $\widehat{S}_{i,j}^{p}$ permet une meilleure séparation spatiale de zones présentant des atténuations différentes, et une meilleure détection des contours. Cela est particulièrement visible sur la partie centrale des images, correspondant aux éléments denses $10_3$ (os). Le recours au spectre du rayonnement primaire permet d'obtenir une valeur de l'atténuation plus conforme à la réalité $\widehat{S}_{i,j}^{p}$.

Ces résultats sont confirmés sur la figure 5F, représentant, un profil central de chacune de ces images, le long de la ligne en pointillés représenté sur ces dernières. Sur cette figure, les indices C, D et E se réfèrent respectivement aux figures 5C, 5D et 5E.

Une reconstruction tomographique de la partie centrale de l'objet représenté sur la figure 1A a été réalisée, en simulant une rotation du dispositif de mesure (source, masque, détecteur) autour de l'objet, l'axe de rotation étant parallèle à l'axe Z, et en réalisant une acquisition par pas angulaire de 1 degré. La source d'irradiation 11 modélisée est un tube à rayons X avec une anode de tungstène, soumis à une tension de 110 kV. Un filtre d'aluminium d'épaisseur 2.5 mm est disposé entre la source 11 et le masque 15. Le détecteur 20 comprend 64 (selon l'axe X) * 680 pixels (selon l'axe Y), chaque pixel comprenant une épaisseur de CdTe de 5 mm. La surface de chaque pixel, dans le plan XY selon lequel s'étend le détecteur, est de 1mm x 1mm. Le détecteur est résolu en énergie, et chaque pixel permet d'obtenir des spectres selon des canaux d'énergie de 10 keV. Le masque 15 utilisé comporte 7 x 68 éléments absorbants régulièrement disposés, de taille 1 mm x 1 mm, d'épaisseur 2 mm.

La reconstruction a été réalisée pour chaque canal d'énergie selon l'algorithme FDK, acronyme de Feldkamp, Davis et Kress, et décrit dans la publication Feldkamp L. et al "Practical cone-beam algorithm", JOSA A,1(6), 612-619, 1984.

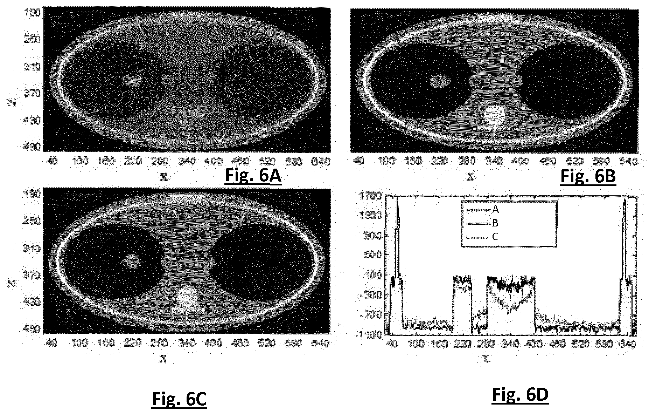

Les figures 6A, 6B et 6C représentent une coupe de l'atténuation, dans un plan transversal XZ, exprimée selon l'unité de Hounsfield, de l'objet reconstruit, respectivement sur la base :

- du spectre du rayonnement total $\mathbf{S}_{i,j}$;

- du spectre du rayonnement primaire simulé $S_{i,j}^{p}$ ;

- du spectre du rayonnement primaire estimé selon l'invention $\widehat{S_{i,j}^{p}}$.

Les abscisses et ordonnées de chacune de ces figures correspondent à des coordonnées respectivement selon l'axe X et l'axe Z.

On remarque une bonne cohérence entre les reconstructions réalisées sur la base du rayonnement primaire simulé et sur la base du rayonnement primaire estimé selon l'invention.

La figure 6D montre un profil horizontal de l'atténuation à la coordonnée z = 340 en considérant respectivement les coupes représentées sur les figures 6A (profil A), 6B (profil B) et 6C (profil C). On remarque la cohérence entre le spectre du rayonnement primaire simulé (profil B) et le spectre du rayonnement primaire estimé selon l'invention (profil C).

L'invention pourra s'appliquer dans des procédés d'imagerie spectrale mettant en oeuvre des rayonnements ionisants, en particulier des rayonnements X ou gamma, pour des applications médicales ou plus généralement, dans le contrôle non destructif d'objets, visant à investiguer la structure interne dudit objet. L'objet peut être, par exemple, un bagage, un produit industriel, un élément de structure d'une installation, par exemple une tuyauterie, un déchet nucléaire, ou un composant aéronautique...

L'invention permet une estimation de la composante primaire d'un rayonnement, limitant ainsi l'influence du rayonnement diffusé. On améliore alors la qualité de l'image obtenue, et en particulier la résolution spatiale. La quantification de l'atténuation produite par l'objet est plus précise, ce qui permet une meilleure estimation de la composition de l'objet examiné. Il en résulte des résultats plus précis, et plus conformes à l'objet examiné.

La mise en oeuvre du procédé est simple et peut s'adapter à des dispositifs existants. De plus, la matrice de passage peut être préalablement établie, ce qui permet une mise en oeuvre du procédé rapide, ne nécessitant pas de temps de calcul élevé. Le procédé est donc adapté à une mise en oeuvre à une cadence industrielle.

**Revendications**

1. Procédé de correction d'un spectre d'un rayonnement électromagnétique ionisant ($I$) transmis par un objet (10),

- l'objet étant disposé entre une source d'irradiation (11) et un détecteur (20), la source d'irradiation étant apte à émettre un rayonnement électromagnétique ionisant, dit rayonnement incident $(I^0)$, vers ledit objet ;
- le détecteur (20) comportant des pixels ($20_{i,j}$), chaque pixel étant configuré pour détecter un rayonnement transmis par l'objet (10) vers le détecteur, et à en acquérir un spectre ($S_{i,j}$), le rayonnement transmis ($I$) comportant un rayonnement diffusé ($I^{diff}$), causé par la diffusion du rayonnement incident $(I^0)$ dans l'objet (10), et un rayonnement primaire ($I^p$);
- un masque (15) étant interposé entre la source (11) et l'objet (10), le masque comportant des éléments absorbants ($15_x$), aptes à atténuer une partie dudit rayonnement incident $(I^0)$ selon une atténuation, la projection de chaque élément absorbant sur le détecteur formant une zone d'ombre ($20_x$), de telle sorte que le détecteur (20) comporte une pluralité de zones d'ombre espacées les unes des autres, chaque zone d'ombre ($20_x$) comportant au moins un pixel ($20_{i,j \in 20_x}$);

le procédé comportant les étapes suivantes :

a) irradiation de l'objet et acquisition, par les pixels du détecteur ($20_{i,j}$), d'un spectre ($S_{i,j}$) du rayonnement ($I$) transmis par l'objet ;

b) sélection d'une région d'analyse ($\Omega$), la région d'analyse comportant au moins un pixel ($20_{i,j \in 20_{x,\Omega}}$) appartenant à une zone d'ombre, dite zone d'ombre d'analyse ($20_{x,\Omega}$) et un pixel ($20_{i,j \notin 20_{x,\Omega}}$) situé à l'extérieur de ladite zone d'ombre d'analyse ($20_{x,\Omega}$);

c) prise en compte d'un spectre $(S_{\Omega}^{diff})$, dit spectre de diffusion, représentant le rayonnement diffusé par l'objet atteignant la région d'analyse ($\Omega$);

d) pour au moins un pixel ($20_{i,j \notin 20_{x,\Omega}}$) de la région d'analyse ($\Omega$), situé dans la zone d'ombre d'analyse ($20_{x,\Omega}$), à partir de l'atténuation du masque (15) et du spectre de diffusion $(S_{\Omega}^{diff})$ pris en compte lors de l'étape c), estimation d'un spectre du rayonnement primaire $(S_{i,j \in 20_{x,\Omega}}^{p})$ transmis par l'objet, en l'absence de masque ;

e) pour au moins un pixel ($20_{i,j \notin 20_{x,\Omega}}$) de la région d'analyse ($\Omega$), situé en dehors de la zone d'ombre d'analyse ($20_{x,\Omega}$), à partir du spectre de diffusion pris en compte de l'étape c), détermination d'un spectre du rayonnement primaire ($S^p_{i,j \notin 20_{x,\Omega}}$) transmis par l'objet ;

f) détermination d'un écart $(\varepsilon | S_{\Omega}^{diff})$ entre les spectres du rayonnement primaire résultant des étapes d) et e), l'écart ainsi déterminé dépendant du spectre de diffusion pris en compte lors de l'étape c) ;

g) répétition des étapes c) à f) en prenant en compte, lors de l'étape c), différents spectres de diffusion $(S_{\Omega}^{diff})$, la répétition des étapes c) à f) visant à déterminer un spectre de diffusion, pris en compte lors de l'étape c), minimisant l'écart déterminé lors de l'étape f) , pour la région d'analyse sélectionnée lors de l'étape b);

h) détermination du spectre de diffusion $(\widehat{S}_{\Omega}^{diff})$ minimisant l'écart déterminé lors de chaque étape f), le spectre de diffusion ainsi déterminé étant associé à la région d'analyse sélectionnée ($\Omega$) lors de l'étape b);

i) réitération des étapes b) à h) en sélectionnant, à chaque itération, différentes régions d'analyse ($\Omega$), de façon à déterminer autant de spectres de diffusion $(\widehat{S}_{\Omega}^{diff})$ que de régions d'analyse ($\Omega$);

j) à partir des spectres de diffusion $(\widehat{S}_{\Omega}^{diff})$ résultant de l'étape i), association d'un spectre de diffusion $(\widehat{S}_{i,j}^{diff})$ à chaque pixel ($20_{i,j}$) et correction d'un spectre ($S_{i,j}$) acquis lors de l'étape a) par au moins un pixel, en fonction du spectre de diffusion associé audit pixel, pour obtenir un spectre corrigé ($S^*_{i,j}$).

**2.** Procédé de correction d'un spectre d'un rayonnement électromagnétique ionisant transmis par un objet (10),

- l'objet étant disposé entre une source d'irradiation (11) et un détecteur (20), la source d'irradiation étant apte à émettre un rayonnement électromagnétique ionisant, dit rayonnement incident $(I^0)$, vers ledit objet ;
- le détecteur (20) comportant des pixels ($20_{i,j}$), chaque pixel étant configuré pour détecter un rayonnement transmis par l'objet (10) vers le détecteur, et à en acquérir un spectre ($\mathbf{S}_{i,j}$), le rayonnement transmis comportant un rayonnement diffusé ($I^{diff}$), causé par la diffusion du rayonnement incident dans l'objet (10), et un rayonnement primaire ($I^p$);
- un masque (15) étant interposé entre la source (11) et l'objet (10), le masque comportant des éléments absorbants ($15_x$), aptes à atténuer une partie dudit rayonnement incident $(I^0)$ selon une atténuation, la projection de chaque élément absorbant sur le détecteur formant une zone d'ombre ($20_x$), de telle sorte que le détecteur (20) comporte une pluralité de zones d'ombre espacées les unes des autres, chaque zone d'ombre ($20_x$) comportant au moins un pixel ($20_{i,j \in 20_x}$);

le procédé comportant les étapes suivantes :

a) irradiation de l'objet et acquisition, par les pixels du détecteur ($20_{i,j}$), d'un spectre ($\mathbf{S}_{i,j}$) du rayonnement transmis par l'objet ;

b) sélection d'une région d'analyse ($\Omega$), la région d'analyse comportant au moins un pixel ($20_{i,j \in 20_{x,\Omega}}$) appartenant à une zone d'ombre, dite zone d'ombre d'analyse ($20_{x,\Omega}$) et un pixel ($20_{i,j \notin 20_{x,\Omega}}$) situé à l'extérieur de ladite zone d'ombre d'analyse ($20_{x,\Omega}$) ;

c) prise en compte d'un spectre $(\mathbf{S}_{\Omega}^{diff})$, dit spectre de diffusion, représentant le rayonnement diffusé par l'objet atteignant la région d'analyse ($\Omega$);

d) pour au moins un pixel ($20_{i,j \notin 20_{x,\Omega}}$) de la région d'analyse ($\Omega$) situé en dehors de la zone d'ombre d'analyse ($20_{x,\Omega}$), à partir de l'atténuation du masque (15) du spectre de diffusion pris en compte de l'étape c), estimation d'un spectre du rayonnement primaire ($\mathbf{S}^p_{i,j \notin 20_{x,\Omega}}$) transmis par l'objet en présence d'un élément absorbant du masque entre la source d'irradiation et ledit pixel;

e) pour au moins un pixel ($20_{i,j \in 20_{x,\Omega}}$) de la région d'analyse ($\Omega$), situé dans la zone d'ombre d'analyse($20_{x,\Omega}$), à partir du spectre de diffusion pris en compte lors de l'étape c), estimation d'un spectre du rayonnement primaire ($\mathbf{S}^p_{i,j \in 20_{x,\Omega}}$) transmis par l'objet ;

f) détermination d'un écart $(\varepsilon | \mathbf{S}_{\Omega}^{diff})$ entre les spectres du rayonnement primaire résultant des étapes d) et e), l'écart ainsi déterminé dépendant du spectre de diffusion pris en compte lors de l'étape c) ;

g) répétition des étapes c) à f) en prenant en compte, lors de l'étape c), différents spectres de diffusion, la répétition des étapes c) à f) visant à déterminer un spectre de diffusion, pris en compte lors de l'étape c), minimisant l'écart déterminé lors de l'étape f), pour la région d'analyse sélectionnée lors de l'étape b) ;

h) détermination du spectre de diffusion $(\widehat{\mathbf{S}}_{\Omega}^{diff})$ minimisant l'écart déterminé lors de chaque étape f), le spectre de diffusion déterminé correspondant à la région d'analyse ($\Omega$) sélectionnée lors de l'étape b);

i) réitération des étapes b) à h) en sélectionnant, à chaque itération, différentes régions d'analyse ($\Omega$), de façon à déterminer autant de spectres de diffusion $(\widehat{\mathbf{S}}_{\Omega}^{diff})$ que de régions d'analyse ;

j) à partir des spectres de diffusion $(\widehat{\mathbf{S}}_{\Omega}^{diff})$ résultant de l'étape i), association d'un spectre de diffusion $(\widehat{\mathbf{S}}_{i,j}^{diff})$ à chaque pixel ($20_{i,j}$) et correction d'un spectre ($\mathbf{S}_{i,j}$) acquis lors de l'étape a) par au moins un pixel, en fonction du spectre de diffusion associé audit pixel, pour obtenir un spectre corrigé ($\mathbf{S}^*_{i,j}$).

**3.** Procédé selon la revendication 1 ou la revendication 2, dans lequel l'étape d) comporte les sous-étapes suivantes :

di) soustraction du spectre de diffusion $(\mathbf{S}_{\Omega}^{diff})$ pris en compte lors de l'étape c) au spectre mesuré, pour le pixel, lors de l'étape a) ;

dii) application d'une matrice de passage (**M, M'**) au spectre résultant de la sous-étape di), la matrice de passage prenant en compte l'atténuation d'un élément absorbant du masque.

**4.** Procédé selon la revendication 3, dans lequel la matrice de passage (**M,M'**) est obtenue au cours d'une phase de calibration, au cours de laquelle l'objet (10) est remplacé par un objet de calibration ($10_k$), la phase de calibration comportant les étapes suivantes :

cal-i) formation d'une matrice diagonale (**C**), dite matrice d'atténuation, chaque terme de la diagonale représentant une atténuation par le masque dans une plage d'énergie ;

cal-ii) irradiation de l'objet de calibration et acquisition, par un pixel du détecteur de calibration du détecteur ($20_{i,jcalib}$), dit pixel de calibration,

- d'un spectre $(S_{i,j calib}^{k})$ du rayonnement transmis par l'objet de calibration ($10_k$) lorsque le masque est interposé entre la source et le détecteur;

- d'un spectre $(S'_{i,j calib}^{k})$ du rayonnement transmis par l'objet de calibration ($10_k$) en l'absence de masque interposé entre la source et le détecteur;

cal-iii) définition d'une matrice de passage (**M**) en appliquant une fonction de déformation (*f*) à une matrice (**C$^{-1}$**) obtenue à partir de la matrice d'atténuation (**C**), la fonction de déformation dépendant de paramètres ($\alpha(p)$, $\beta(p)$)*;*

cal-iv) application de la matrice de passage (**M**) au spectre du rayonnement transmis par l'objet de calibration $(S_{i,j calib}^{k})$ en présence du masque ;

cal-v) réitération des étapes cal-ii) à cal-iv) pour différents objets de calibration ;

cal-vi) obtention des paramètres de la fonction minimisant un écart entre les spectres résultant de l'étape cal-iv) à chaque spectre $(S'_{i,j calib}^{k})$ du rayonnement transmis par l'objet de calibration en l'absence du masque interposé entre la source et le détecteur.

5. Procédé selon l'une quelconque des revendications précédentes, dans lequel l'étape j) comporte pour chaque pixel ($20_{i,j \in 20_x}$) situé à l'intérieur d'une zone d'ombre ($20_x$), les sous-étapes suivantes :

ji) détermination d'un spectre primaire, dit spectre primaire atténué $(S_{i,j \in 20_{x,\Omega}}^{p,x})$, à partir du spectre acquis, par le pixel, lors de l'étape a) en fonction du spectre de diffusion $(\widehat{S}_{i,j}^{diff})$ associé audit pixel lors de l'étape j);

jii) correction du spectre résultant de la sous-étape ji) en prenant en compte l'atténuation du masque, pour estimer un spectre primaire ($S^{p}_{i,j \in 20_{x,\Omega}}$) en l'absence de masque.

6. Procédé selon l'une quelconque des revendications précédentes, dans lequel l'étape j) comporte, pour chaque pixel ($20_{i,j \notin 20_x}$) situé à l'extérieur d'une zone d'ombre ($20_x$), une soustraction du spectre de diffusion $(\widehat{S}_{i,j}^{diff})$ associé audit pixel lors de l'étape i) au spectre acquis, par ledit pixel, lors de l'étape a).

7. Procédé selon l'une quelconque des revendications précédentes, comportant, suite à l'étape i), un lissage entre les spectres de diffusion $(\widetilde{S}_{i,j}^{diff})$ associés respectivement à des pixels différents.

8. Procédé selon l'une quelconque des revendications différentes, dans lequel à chaque région d'analyse ($\Omega_{i,j}$) est associé un pixel ($20_{i,j}$), le procédé étant mis en oeuvre sur les régions d'analyse associées à tout ou partie des pixels du détecteur.

9. Procédé selon l'une quelconque des revendications précédentes, dans lequel chaque élément absorbant ($15_x$) est apte à absorber entre 5 % et 80 % du rayonnement auquel il est exposé, dans une bande d'énergie prédéterminée.

10. Procédé selon l'une quelconque des revendications précédentes, dans lequel, le masque (15) s'étendant selon une surface, chaque élément absorbant est distant d'un autre élément absorbant d'une distance inférieure à 1 cm.

11. Procédé selon l'une quelconque des revendications précédentes, dans lequel les étapes a) à j) sont mises en oeuvre selon une pluralité de configurations (*C*), à chaque configuration étant associée une position du détecteur et de la source par rapport à l'objet, de façon à obtenir, dans chaque configuration, pour une pluralité de pixels, un spectre corrigé ($S^*_{i,j}$), les spectres corrigés de chaque configuration étant utilisés pour réaliser une reconstruction tomographique de l'objet (10).

12. Procédé selon l'une quelconque des revendications précédentes, dans lequel les étapes a) à j) sont complétées par les étapes suivantes :

    k) sélection d'au moins une énergie ($E$) ou une plage d'énergie ($\Delta E$);
    l) réalisation d'une image, dont chaque pixel comprend une donnée obtenue à partir d'un spectre corrigé, associé à un pixel ($20_{i,j}$) du détecteur, à ladite énergie ($E$) ou dans ladite plage d'énergie ($\Delta E$).

13. Procédé selon la revendication 12, dans lequel lors de l'étape l) chaque pixel de l'image comporte une information relative à une intégrale ou à une moyenne dudit spectre corrigé ($S^*_{i,j}$) dans ladite plage d'énergie ($\Delta E$).

14. Procédé selon l'une quelconque des revendications précédentes, dans lequel le masque (15) est interposé entre la source d'irradiation (11) et l'objet (10).

15. Support d'enregistrement d'informations, comportant des instructions pour l'exécution des étapes b) à j) du procédé selon l'une quelconque des revendications 1 à 14, à partir des spectres acquis lors de l'étape a), ces instructions étant exécutées par un microprocesseur.

16. Dispositif pour l'acquisition de spectres d'un rayonnement transmis par un objet (10) comportant :

    ▪ une source d'irradiation (11), apte à émettre un rayonnement électromagnétique ionisant, dit rayonnement incident ($I^0$), vers ledit objet (10);
    ▪ un détecteur (20) comportant des pixels ($20_{i,j}$), chaque pixel étant configuré pour détecter un rayonnement (10) transmis par l'objet vers le détecteur, et à en acquérir un spectre ($S_{i,j}$);
    ▪ un masque (15), apte à être interposé entre la source (11) et l'objet (10), ledit masque comportant des éléments absorbants ($15_x$), aptes à absorber une partie dudit rayonnement incident ($I^0$), et dont une projection sur le détecteur définit des zones d'ombres ($20x$) distantes les unes des autres ;
    ▪ un processeur (21), configuré pour recevoir des spectres acquis par chaque pixel, et à mettre en oeuvre les étapes b) à j) du procédé objet de l'une quelconque des revendications 1 à 14.

**Patentansprüche**

1. Verfahren zur Korrektur eines Spektrums einer ionisierenden elektromagnetischen Strahlung ($I$), die von einem Objekt (10) transmittiert wird,

    - wobei das Objekt zwischen einer Bestrahlungsquelle (11) und einem Detektor (20) angeordnet ist, wobei die Bestrahlungsquelle in der Lage ist, eine ionisierende elektromagnetische Strahlung, einfallende Strahlung ($I^0$) genannt, in Richtung des Objekts zu emittieren;
    - wobei der Detektor (20) Pixel ($20_{i,j}$) aufweist, wobei jedes Pixel dafür ausgelegt ist, eine von dem Objekt (10) in Richtung des Detektors transmittierte Strahlung zu detektieren und ein Spektrum ($S_{i,j}$) derselben zu erfassen, wobei die transmittierte Strahlung ($I$) eine Streustrahlung ($I$diff), die durch die Streuung der einfallenden Strahlung ($I^0$) im Objekt (10) verursacht wird, und eine Primärstrahlung ($I$p) umfasst;
    - wobei eine Maske (15) zwischen der Quelle (11) und dem Objekt (10) angeordnet ist, wobei die Maske absorbierende Elemente ($15_x$) aufweist, die in der Lage sind, einen Teil der einfallenden Strahlung ($I^0$) gemäß einer Dämpfung zu dämpfen, wobei die Projektion jedes absorbierenden Elements auf den Detektor einen Schattenbereich ($20_x$) bildet, so dass der Detektor (20) mehrere voneinander beabstandete Schattenbereiche aufweist, wobei jeder Schattenbereich ($20_x$) wenigstens ein Pixel ($20_{i,j\in20_x}$) aufweist;

    wobei das Verfahren die folgenden Schritte umfasst:

    a) Bestrahlung des Objekts und Erfassung, durch die Pixel des Detektors ($20_{i,j}$), eines Spektrums ($S_{i,j}$) der von dem Objekt transmittierten Strahlung ($I^0$);
    b) Auswahl eines Analysegebiets ($\Omega$), wobei das Analysegebiet wenigstens ein Pixel ($20_{i,j\in20_{x,\Omega}}$), das einem Schattenbereich angehört, Analyseschattenbereich ($20_{x,\Omega}$) genannt, und ein Pixel ($20_{i,j\notin20_{x,\Omega}}$), das sich außer-

halb des Analyseschattenbereichs ($20_{x,\Omega}$) befindet, umfasst;

c) Berücksichtigung eines Spektrums $\left(S_\Omega^{diff}\right)$, Streuspektrum genannt, welches die durch das Objekt gestreute Strahlung repräsentiert, die das Analysegebiet ($\Omega$) erreicht;

d) für wenigstens ein Pixel ($20_{i,j \in 20_{x,\Omega}}$) des Analysegebiets ($\Omega$), das sich im Analyseschattenbereich ($20_{x,\Omega}$) befindet, ausgehend von der Dämpfung der Maske (15) und von dem in Schritt c) berücksichtigten Streuspektrum $\left(S_\Omega^{diff}\right)$, Schätzung eines Spektrums der Primärstrahlung ($S^p_{i,j \in 20_{x,\Omega}}$), die von dem Objekt bei Nichtvorhandensein einer Maske transmittiert wird;

e) für wenigstens ein Pixel ($20_{i,j \notin 20_{x,\Omega}}$) des Analysegebiets ($\Omega$), das sich außerhalb des Analyseschattenbereichs ($20_{x,\Omega}$) befindet, ausgehend von dem berücksichtigten Streuspektrum von Schritt c), Bestimmung eines Spektrums der Primärstrahlung ($S^p_{i,j \notin 20_{x,\Omega}}$), die von dem Objekt transmittiert wird;

f) Bestimmung einer Abweichung $\left(\varepsilon | S_\Omega^{diff}\right)$ zwischen den Spektren der Primärstrahlung, die aus den Schritten d) und e) resultieren, wobei die so bestimmte Abweichung von dem in Schritt c) berücksichtigten Streuspektrum abhängt;

g) Wiederholung der Schritte c) bis f) unter Berücksichtigung, in Schritt c), verschiedener Streuspektren $\left(S_\Omega^{diff}\right)$, wobei die Wiederholung der Schritte c) bis f) darauf abzielt, ein in Schritt c) berücksichtigtes Streuspektrum, das die in Schritt f) bestimmte Abweichung minimiert, für das in Schritt b) ausgewählte Analysegebiet zu bestimmen;

h) Bestimmung des Streuspektrums $\left(\widehat{S}_\Omega^{diff}\right)$, das die in jedem Schritt f) bestimmte Abweichung minimiert, wobei das so bestimmte Streuspektrum dem in Schritt b) ausgewählten Analysegebiet ($\Omega$) zugeordnet wird;

i) iterative Wiederholung der Schritte b) bis h) mit Auswahl, in jeder Iteration, verschiedener Analysegebiete ($\Omega$), um ebenso viele Streuspektren $\left(\widehat{S}_\Omega^{diff}\right)$ wie Analysegebiete ($\Omega$) zu bestimmen;

j) ausgehend von den aus Schritt i) resultierenden Streuspektren $\left(\widehat{S}_\Omega^{diff}\right)$, Zuordnung eines Streuspektrums $\left(\widehat{S}_{i,j}^{diff}\right)$ zu jedem Pixel ($20_{i,j}$) und Korrektur eines in Schritt a) erfassten Spektrums ($S_{i,j}$) durch wenigstens ein Pixel in Abhängigkeit von dem diesem Pixel zugeordneten Streuspektrum, um ein korrigiertes Spektrum ($S^*_{i,j}$) zu erhalten.

2. Verfahren zur Korrektur eines Spektrums einer ionisierenden elektromagnetischen Strahlung, die von einem Objekt (10) transmittiert wird,

- wobei das Objekt zwischen einer Bestrahlungsquelle (11) und einem Detektor (20) angeordnet ist, wobei die Bestrahlungsquelle in der Lage ist, eine ionisierende elektromagnetische Strahlung, einfallende Strahlung ($I^0$) genannt, in Richtung des Objekts zu emittieren;
- wobei der Detektor (20) Pixel ($20_{i,j}$) aufweist, wobei jedes Pixel dafür ausgelegt ist, eine von dem Objekt (10) in Richtung des Detektors transmittierte Strahlung zu detektieren und ein Spektrum ($S_{i,j}$) derselben zu erfassen, wobei die transmittierte Strahlung eine Streustrahlung ($I^{diff}$), die durch die Streuung der einfallenden Strahlung ($I^0$) im Objekt (10) verursacht wird, und eine Primärstrahlung ($I^p$) umfasst;
- wobei eine Maske (15) zwischen der Quelle (11) und dem Objekt (10) angeordnet ist, wobei die Maske absorbierende Elemente ($15_x$) aufweist, die in der Lage sind, einen Teil der einfallenden Strahlung ($I^0$) gemäß einer Dämpfung zu dämpfen, wobei die Projektion jedes absorbierenden Elements auf den Detektor einen Schattenbereich ($20_x$) bildet, so dass der Detektor (20) mehrere voneinander beabstandete Schattenbereiche aufweist, wobei jeder Schattenbereich ($20_x$) wenigstens ein Pixel ($20_{i,j \in 20_x}$) aufweist;

wobei das Verfahren die folgenden Schritte umfasst:

a) Bestrahlung des Objekts und Erfassung, durch die Pixel des Detektors ($20_{i,j}$), eines Spektrums ($S_{i,j}$) der von dem Objekt transmittierten Strahlung;
b) Auswahl eines Analysegebiets ($\Omega$), wobei das Analysegebiet wenigstens ein Pixel ($20_{i,j \in 20_{x,\Omega}}$), das einem

Schattenbereich angehört, Analyseschattenbereich ($20_{x,\Omega}$) genannt, und ein Pixel ($20_{i,j \notin 20x,\Omega}$), das sich außerhalb des Analyseschattenbereichs ($20_{x,\Omega}$) befindet, umfasst;

c) Berücksichtigung eines Spektrums $\left(S_\Omega^{diff}\right)$, Streuspektrum genannt, welches die durch das Objekt gestreute Strahlung repräsentiert, die das Analysegebiet ($\Omega$) erreicht;

d) für wenigstens ein Pixel ($20_{i,j \notin 20x,\Omega}$) des Analysegebiets ($\Omega$), das sich außerhalb des Analyseschattenbereichs ($20_{x,\Omega}$) befindet, ausgehend von der Dämpfung der Maske (15) des in Schritt c) berücksichtigten Streuspektrums, Schätzung eines Spektrums der Primärstrahlung ($Sp_{i,j \notin 20x,\Omega}$), die von dem Objekt bei Vorhandensein eines absorbierenden Elements der Maske zwischen der Bestrahlungsquelle und dem Pixel transmittiert wird;

e) für wenigstens ein Pixel ($20_{i,j \in 20x,\Omega}$) des Analysegebiets ($\Omega$), das sich im Analyseschattenbereich ($20_{x,\Omega}$) befindet, ausgehend von dem in Schritt c) berücksichtigten Streuspektrum, Schätzung eines Spektrums der Primärstrahlung ($Sp_{i,j \in 20x,\Omega}$), die von dem Objekt transmittiert wird;

f) Bestimmung einer Abweichung $\left(\varepsilon | S_\Omega^{diff}\right)$ zwischen den Spektren der Primärstrahlung, die aus den Schritten d) und e) resultieren, wobei die so bestimmte Abweichung von dem in Schritt c) berücksichtigten Streuspektrum abhängt;

g) Wiederholung der Schritte c) bis f) unter Berücksichtigung, in Schritt c), verschiedener Streuspektren, wobei die Wiederholung der Schritte c) bis f) darauf abzielt, ein in Schritt c) berücksichtigtes Streuspektrum, das die in Schritt f) bestimmte Abweichung minimiert, für das in Schritt b) ausgewählte Analysegebiet zu bestimmen;

h) Bestimmung des Streuspektrums $\left(\hat{S}_\Omega^{diff}\right)$, das die in jedem Schritt f) bestimmte Abweichung minimiert, wobei das bestimmte Streuspektrum dem in Schritt b) ausgewählten Analysegebiet ($\Omega$) entspricht;

i) iterative Wiederholung der Schritte b) bis h) mit Auswahl, in jeder Iteration, verschiedener Analysegebiete ($\Omega$), um ebenso viele Streuspektren $\left(\hat{S}_\Omega^{diff}\right)$ wie Analysegebiete zu bestimmen;

j) ausgehend von den aus Schritt i) resultierenden Streuspektren $\left(\hat{S}_\Omega^{diff}\right)$, Zuordnung eines Streuspektrums $\left(\hat{S}_{i,j}^{diff}\right)$ zu jedem Pixel ($20_{i,j}$) und Korrektur eines in Schritt a) erfassten Spektrums ($S_{i,j}$) durch wenigstens ein Pixel in Abhängigkeit von dem diesem Pixel zugeordneten Streuspektrum, um ein korrigiertes Spektrum ($S^*_{i,j}$) zu erhalten.

3. Verfahren nach Anspruch 1 oder Anspruch 2, wobei Schritt d) die folgenden Teilschritte umfasst:

di) Subtraktion des in Schritt c) berücksichtigten Streuspektrums $\left(S_\Omega^{diff}\right)$ von dem für das Pixel in Schritt a) gemessenen Spektrum;

dii) Anwendung einer Übergangsmatrix (**M, M'**) auf das aus dem Teilschritt di) resultierende Spektrum, wobei die Übergangsmatrix die Dämpfung eines absorbierenden Elements der Maske berücksichtigt.

4. Verfahren nach Anspruch 3, wobei die Übergangsmatrix (**M, M'**) während einer Kalibrierungsphase erhalten wird, in welcher das Objekt (10) durch ein Kalibrierungsobjekt ($10_k$) ersetzt wird, wobei die Kalibrierungsphase die folgenden Schritte umfasst:

cal-i) Bildung einer Diagonalmatrix (**C**), Dämpfungsmatrix genannt, wobei jeder Term der Diagonale eine Dämpfung durch die Maske in einem Energiebereich repräsentiert;

cal-ii) Bestrahlung des Kalibrierungsobjekts und Erfassung, für ein Pixel des Kalibrierungsdetektors des Detektors ($20_{i,jcalib}$), Kalibrierungspixel genannt,

• eines Spektrums $\left(S_{i,jcalib}^{k}\right)$ der von dem Kalibrierungsobjekt ($10_k$) transmittierten Strahlung, wenn die Maske zwischen der Quelle und dem Detektor angeordnet ist;

• eines Spektrums $\left(S'^{k}_{i,jcalib}\right)$ der von dem Kalibrierungsobjekt ($10_k$) bei Nichtvorhandensein der zwischen der Quelle und dem Detektor angeordneten Maske transmittierten Strahlung;

cal-iii) Definition einer Übergangsmatrix (**M**) unter Anwendung einer Deformationsfunktion (f) auf eine aus der Dämpfungsmatrix (**C**) erhaltene Matrix (**C**$^{-1}$), wobei die Deformationsfunktion von Parametern ($\alpha(p)$, $\beta(p)$) ab-

hängt;

cal-iv) Anwendung der Übergangsmatrix (**M**) auf das Spektrum der Strahlung $\left(\boldsymbol{S}^{k}_{i,j\,calib}\right)$, die von dem Kalibrierungsobjekt bei Vorhandensein der Maske transmittiert wird;

cal-v) iterative Wiederholung der Schritte cal-ii) bis cal-iv) für verschiedene Kalibrierungsobjekte;

cal-vi) Gewinnung der Parameter der Funktion, die eine Abweichung zwischen den aus Schritt cal-iv) resultierenden Spektren und jedem Spektrum $\left(\boldsymbol{S'}^{k}_{i,j\,calib}\right)$ der von dem Kalibrierungsobjekt bei Nichtvorhandensein der zwischen der Quelle und dem Detektor angeordneten Maske transmittierten Strahlung minimieren.

**5.** Verfahren nach einem der vorhergehenden Ansprüche, wobei Schritt j) für jedes Pixel ($20_{i,j\in 20_x}$), das sich innerhalb eines Schattenbereichs ($20_x$) befindet, die folgenden Teilschritte umfasst:

ji) Bestimmung eines Primärspektrums, gedämpftes Primärspektrum $\left(\boldsymbol{S}^{p,x}_{i,j\in 20_{x,\Omega}}\right)$ genannt, aus dem durch das Pixel in Schritt a) erfassten Spektrum in Abhängigkeit von dem Streuspektrum $\left(\widehat{\boldsymbol{S}}^{diff}_{i,j}\right)$, das dem Pixel in Schritt j) zugeordnet wird;

jii) Korrektur des aus dem Teilschritt ji) resultierenden Spektrums unter Berücksichtigung der Dämpfung der Maske, um ein Primärspektrum ($S^p_{i,j\in 20_{x,\Omega}}$) bei Nichtvorhandensein einer Maske zu schätzen.

**6.** Verfahren nach einem der vorhergehenden Ansprüche, wobei Schritt j) für jedes Pixel ($20_{i,j\notin 20_x}$), das sich außerhalb eines Schattenbereichs ($20_x$) befindet, eine Subtraktion des Streuspektrums $\left(\widehat{\boldsymbol{S}}^{diff}_{i,j}\right)$, das dem Pixel in Schritt i) zugeordnet wurde, von dem durch das Pixel in Schritt a) erfassten Spektrum umfasst.

**7.** Verfahren nach einem der vorhergehenden Ansprüche, welches im Anschluss an Schritt i) eine Glättung zwischen den Streuspektren $\left(\tilde{\boldsymbol{S}}^{diff}_{i,j}\right)$, die jeweils verschiedenen Pixeln zugeordnet sind, umfasst.

**8.** Verfahren nach einem der verschiedenen Ansprüche, wobei jedem Analysegebiet ($\Omega_{i,j}$) ein Pixel ($20_{i,j}$) zugeordnet wird, wobei das Verfahren in den Analysegebieten durchgeführt wird, die jedem oder einem Teil der Pixel des Detektors zugeordnet sind.

**9.** Verfahren nach einem der vorhergehenden Ansprüche, wobei jedes absorbierende Element ($15_x$) in der Lage ist, zwischen 5 % und 80 % der Strahlung, welcher es ausgesetzt ist, in einem vorbestimmten Energieband zu absorbieren.

**10.** Verfahren nach einem der vorhergehenden Ansprüche, wobei, wenn die Maske (15) sich entlang einer Fläche erstreckt, jedes absorbierende Element von einem anderen absorbierenden Element einen Abstand hat, der kleiner als 1 cm ist.

**11.** Verfahren nach einem der vorhergehenden Ansprüche, wobei die Schritte a) bis j) für mehrere Konfigurationen (*C*) ausgeführt werden, wobei jeder Konfiguration eine Position des Detektors und der Quelle in Bezug auf das Objekt zugeordnet ist, um so in jeder Konfiguration für mehrere Pixel ein korrigiertes Spektrum ($\boldsymbol{S}^*_{i,j}$) zu erhalten, wobei die korrigierten Spektren jeder Konfiguration verwendet werden, um eine tomographische Rekonstruktion des Objekts (10) durchzuführen.

**12.** Verfahren nach einem der vorhergehenden Ansprüche, wobei die Schritte a) bis j) um die folgenden Schritte ergänzt werden:

k) Auswahl wenigstens einer Energie (*E*) oder eines Energiebereichs ($\Delta E$);
l) Herstellung eines Bildes, von dem jedes Pixel ein Datenelement umfasst, das aus einem korrigierten Spektrum erhalten wurde, das einem Pixel ($20_{i,j}$) des Detektors zugeordnet ist, bei der Energie (*E*) oder im Energiebereich ($\Delta E$).

**13.** Verfahren nach Anspruch 12, wobei in Schritt l) jedes Pixel des Bildes eine Information in Bezug auf ein Integral oder einen Mittelwert des korrigierten Spektrums ($S^*_{i,j}$) im Energiebereich ($\Delta E$) umfasst.

14. Verfahren nach einem der vorhergehenden Ansprüche, wobei die Maske (15) zwischen der Bestrahlungsquelle (11) und dem Objekt (10) angeordnet ist.

15. Informationsaufzeichnungsmedium, welches Anweisungen zur Ausführung der Schritte b) bis j) des Verfahrens nach einem der Ansprüche 1 bis 14 ausgehend von den in Schritt a) erfassten Spektren umfasst, wobei diese Anwendungen von einem Mikroprozessor ausgeführt werden.

16. Vorrichtung zur Erfassung von Spektren einer Strahlung, die von einem Objekt (10) transmittiert wird, umfassend:

   • eine Bestrahlungsquelle (11), welche in der Lage ist, eine ionisierende elektromagnetische Strahlung, einfallende Strahlung ($I^0$) genannt, in Richtung des Objekts (10) zu emittieren;
   • einen Detektor (20), welcher Pixel ($20_{i,j}$) aufweist, wobei jedes Pixel dafür ausgelegt ist, eine von dem Objekt (10) in Richtung des Detektors transmittierte Strahlung zu detektieren und ein Spektrum ($S_{i,j}$) derselben zu erfassen;
   • eine Maske (15), die geeignet ist, zwischen der Quelle (11) und dem Objekt (10) angeordnet zu werden, wobei die Maske absorbierende Elemente ($15_x$) aufweist, die in der Lage sind, einen Teil der einfallenden Strahlung ($I^0$) zu absorbieren, und wobei eine Projektion von ihr auf den Detektor Schattenbereiche ($20_x$) definiert, die voneinander beabstandet sind;
   • einen Prozessor (21), der dafür ausgelegt ist, von jedem Pixel erfasste Spektren zu empfangen und die Schritte b) bis j) des Verfahrens auszuführen, welches Gegenstand eines der Ansprüche 1 bis 14 ist.

**Claims**

1. Method for correcting a spectrum of ionizing electromagnetic radiation ($I$) transmitted by an object (10),

   - the object being placed between a radiation source (11) and a detector (20), the radiation source being able to emit ionizing electromagnetic radiation, called incident radiation ($I^0$), toward said object;
   - the detector (20) comprising pixels ($20_{i,j}$), each pixel being configured to detect radiation transmitted by the object (10) toward the detector, and to acquire a spectrum ($S_{i,j}$) thereof, the transmitted radiation ($I$) comprising scattered radiation ($I^{diff}$), caused by the scatter of the incident radiation ($I^0$) in the object (10), and primary radiation ($I^p$);
   - a mask (15) being interposed between the source (11) and the object (10), the mask comprising absorbing elements ($15_x$), which are able to attenuate a portion of said incident radiation ($I^0$) with an attenuation, the projection of each absorbing element onto the detector forming a shadow zone ($20_x$), such that the detector (20) comprises a plurality of shadow zones that are spaced apart from one another, each shadow zone ($20_x$) comprising at least one pixel ($20_{i,j \in 20_x}$);

   the method comprising the following steps:

   a) irradiating the object and acquiring, with the pixels ($20_{i,j}$) of the detector, a spectrum ($S_{i,j}$) of the radiation ($I$) transmitted by the object;
   b) selecting an analysis region ($\Omega$), the analysis region comprising at least one pixel ($20_{i,j \in 20_{x,\Omega}}$) belonging to a shadow zone, called the analysis shadow zone ($20_{x,\Omega}$), and one pixel ($20_{i,j \notin 20_{x,\Omega}}$) located outside of said analysis shadow zone ($20_{x,\Omega}$);
   c) taking into account a spectrum $(S_\Omega^{diff})$, called the scattering spectrum, representing the radiation scattered by the object reaching the analysis region ($\Omega$);
   d) for at least one pixel ($20_{i,j \in 20_{x,\Omega}}$) of the analysis region ($\Omega$), which pixel is located in the analysis shadow zone ($20_{x,\Omega}$), on the basis of the attenuation of the mask (15) and of the scattering spectrum $(S_\Omega^{diff})$ taken into account in step c), estimating a spectrum of the primary radiation $(S_{i,j \in 20_{x,\Omega}}^p)$ transmitted by the object, in the absence of mask;
   e) for at least one pixel ($20_{i,j \in 20_{x,\Omega}}$) of the analysis region ($\Omega$), which pixel is located outside of the analysis

27

shadow zone $(20_{x,\Omega})$, on the basis of the scattering spectrum taken into account in step c), determining a spectrum $\left(S^{p}_{i,j \notin 20_{x,\Omega}}\right)$ of the primary radiation transmitted by the object;

f) determining a discrepancy $\left(\varepsilon|S^{diff}_{\Omega}\right)$ between the spectra of the primary radiation resulting from steps d) and e), the discrepancy thus determined depending on the scattering spectrum taken into account in step c);

g) repeating steps c) to f) while taking into account, in step c), various scattering spectra $\left(S^{diff}_{\Omega}\right)$, the repetition of steps c) to f) aiming to determine a scattering spectrum, taken into account in step c), minimizing the discrepancy determined in step f), for the analysis region selected in step b);

h) determining the scattering spectrum $\left(\widehat{S}^{diff}_{\Omega}\right)$ minimizing the discrepancy determined in each step f), the scattering spectrum thus determined being associated with the analysis region ($\Omega$) selected in step b);

i) reiterating steps b) to h) while selecting, in each iteration, various analysis regions ($\Omega$), so as to determine as many scattering spectra $\left(\widehat{S}^{diff}_{\Omega}\right)$ as there are analysis regions ($\Omega$);

j) based on the scattering spectra $\left(\widehat{S}^{diff}_{\Omega}\right)$ resulting from step i), associating one scattering spectrum $\left(\widehat{S}^{diff}_{i,j}\right)$ with each pixel $(20_{i,j})$ and correcting a spectrum ($S_{i,j}$) acquired in step a) with a least one pixel, depending on the scattering spectrum associated with said pixel, in order to obtain a corrected spectrum ($S^{*}_{i,j}$).

2.  Method for correcting a spectrum of ionizing electromagnetic radiation transmitted by an object (10),

- the object being placed between a radiation source (11) and a detector (20), the radiation source being able to emit ionizing electromagnetic radiation, called incident radiation ($I^0$), toward said object;
- the detector (20) comprising pixels $(20_{i,j})$, each pixel being configured to detect radiation transmitted by the object (10) toward the detector, and to acquire a spectrum ($S_{i,j}$) thereof, the transmitted radiation comprising scattered radiation ($I^{diff}$), caused by the scatter of the incident radiation in the object (10), and primary radiation ($I^p$);
- a mask (15) being interposed between the source (11) and the object (10), the mask comprising absorbing elements $(15_x)$, which are able to attenuate a portion of said incident radiation ($I^0$) with an attenuation, the projection of each absorbing element onto the detector forming a shadow zone $(20_x)$, such that the detector (20) comprises a plurality of shadow zones that are spaced apart from one another, each shadow zone $(20_x)$ comprising at least one pixel $(20_{i,j \in 20_x})$;

the method comprising the following steps:

a) irradiating the object and acquiring, with the pixels $(20_{i,j})$ of the detector, a spectrum ($S_{i,j}$) of the radiation transmitted by the object;
b) selecting an analysis region ($\Omega$), the analysis region comprising at least one pixel $(20_{i,j \in 20_{x,\Omega}})$ belonging to a shadow zone, called the analysis shadow zone $(20_{x,\Omega})$, and one pixel $(20_{i,j \notin 20_{x,\Omega}})$ located outside of said analysis shadow zone $(20_{x,\Omega})$;

c) taking into account a spectrum $\left(S^{diff}_{\Omega}\right)$, called the scattering spectrum, representing the radiation scattered by the object reaching the analysis region ($\Omega$);
d) for at least one pixel $(20_{i,j \notin 20_{x,\Omega}})$ of the analysis region ($\Omega$), which pixel is located outside of the analysis shadow zone $(20_{x,\Omega})$, on the basis of the attenuation of the mask (15) of the scattering spectrum taken into account in step c), estimating a spectrum of the primary radiation $\left(S^{p}_{i,j \notin 20_{x,\Omega}}\right)$ transmitted by the object, in the presence of an absorbing element of the mask between the radiation source and said pixel;
e) for at least one pixel $(20_{i,j \in 20_{x,\Omega}})$ of the analysis region ($\Omega$), which pixel is located in the analysis shadow zone $(20_{x,\Omega})$, on the basis of the scattering spectrum taken into account in step c), estimating a spectrum $\left(S^{p}_{i,j \in 20_{x,\Omega}}\right)$ of the primary radiation transmitted by the object;

f) determining a discrepancy $\left(\varepsilon | S_\Omega^{diff}\right)$ between the spectra of the primary radiation resulting from steps d) and e), the discrepancy thus determined depending on the scattering spectrum taken into account in step c);

g) repeating steps c) to f) while taking into account, in step c), various scattering spectra, the repetition of steps c) to f) aiming to determine a scattering spectrum, taken into account in step c), minimizing the discrepancy determined in step f), for the analysis region selected in step b);

h) determining the scattering spectrum $\left(\widehat{S}_\Omega^{diff}\right)$ minimizing the discrepancy determined in each step f), the determined scattering spectrum corresponding to the analysis region ($\Omega$) selected in step b);

i) reiterating steps b) to h) while selecting, in each iteration, various analysis regions ($\Omega$), so as to determine as many scattering spectra $\left(\widehat{S}_\Omega^{diff}\right)$ as there are analysis regions;

j) based on the scattering spectra $\left(\widehat{S}_\Omega^{diff}\right)$ resulting from step i), associating one scattering spectrum $\left(\widehat{S}_{i,j}^{diff}\right)$ with each pixel ($20_{i,j}$) and correcting a spectrum ($S_{i,j}$) acquired in step a) with a least one pixel, depending on the scattering spectrum associated with said pixel, in order to obtain a corrected spectrum ($S^*_{i,j}$).

3. Method according to Claim 1 or Claim 2, wherein step d) comprises the following substeps:

di) subtracting the scattering spectrum $\left(S_\Omega^{diff}\right)$ taken into account in step c) from the spectrum measured, for the pixel, in step a);

dii) applying a change-of-basis matrix ($M$, $M'$) to the spectrum resulting from substep di), the change-of-basis matrix taking into account the attenuation of an absorbing element of the mask.

4. Method according to Claim 3, wherein the change-of-basis matrix ($M$, $M'$) is obtained in a calibration phase, in which the object (10) is replaced by a calibration object ($10_k$), the calibration phase comprising the following steps:

cal-i) forming a diagonal matrix ($C$), called the attenuation matrix, each term of the diagonal representing an attenuation by the mask in an energy range;

cal-ii) irradiating the calibration object and acquiring, with a pixel ($20_{i,jcalib}$) of the detector for calibrating the detector, called the calibration pixel,

- a spectrum $\left(S_{i,j^{calib}}^k\right)$ of the radiation transmitted by the calibration object ($10_k$) when the mask is interposed between the source and the detector;

- a spectrum $\left(S'^k_{i,j^{calib}}\right)$ of the radiation transmitted by the calibration object ($10_k$) in the absence of mask interposed between the source and the detector;

cal-iii) defining a change-of-basis matrix ($M$) by applying a deformation function ($f$) to a matrix ($C^{-1}$) obtained from the attenuation matrix ($C$), the deformation function depending on parameters ($\alpha(p)$, $\beta(p)$);

cal-iv) applying the change-of-basis matrix ($M$) to the spectrum $\left(S_{i,j^{calib}}^k\right)$ of the radiation transmitted by the calibration object in the presence of the mask;

cal-v) reiterating steps cal-ii) to cal-iv) for various calibration objects;

cal-vi) obtaining parameters of the function minimizing a discrepancy between the spectra resulting from step cal-iv) and each spectrum $\left(S'^k_{i,j^{calib}}\right)$ of the radiation transmitted by the calibration object in the absence of the mask interposed between the source and the detector.

5. Method according to any one of the preceding claims, wherein step j) comprises for each pixel ($20_{i,j \in 20_x}$) located inside a shadow zone ($20_x$), the following substeps:

ji) determining a primary spectrum, called the attenuated primary spectrum $\left(S_{i,j \in 20_x,\Omega}^{p,x}\right),$ from the spectrum acquired, with the pixel, in step a) depending on the scattering spectrum $(\widehat{S}_{i,j}^{diff})$ associated with said pixel in step j);

jii) correcting the spectrum resulting from substep ji) by taking into account the

attenuation of the mask, in order to estimate a primary spectrum $(S_{i,j\,\in\,20_{x,\Omega}}^{p})$ in the absence of mask.

6. Method according to any one of the preceding claims, wherein step j) comprises, for each pixel ($20_{i,j\notin20_x}$) located outside of a shadow zone ($20_x$), subtracting the scattering spectrum $(\widehat{S}_{i,j}^{diff})$ associated with said pixel in step i) from the spectrum acquired, with said pixel, in step a).

7. Method according to any one of the preceding claims, comprising, following step i), smoothing between the scattering spectra $(\tilde{S}_{i,j}^{diff})$ respectively associated with different pixels.

8. Method according to any one of the different claims, wherein one pixel ($20_{i,j}$) is associated with each analysis region ($\Omega_{i,j}$), the method being implemented on analysis regions associated with all or some of the pixels of the detector.

9. Method according to any one of the preceding claims, wherein each absorbing element ($15_x$) is able to absorb between 5% and 80% of the radiation to which it is exposed, in a predefined energy band.

10. Method according to any one of the preceding claims, wherein the mask (15) occupies an area, each absorbing element being distant from another absorbing element by a distance smaller than 1 cm.

11. Method according to any one of the preceding claims, wherein steps a) to j) are implemented in a plurality of configurations (C), each configuration being associated with one position of the detector and of the source with respect to the object, so as to obtain, in each configuration, for a plurality of pixels, a corrected spectrum ($S^*_{i,j}$), the corrected spectra of each configuration being used to carry out a tomographic reconstruction of the object (10).

12. Method according to any one of the preceding claims, wherein steps a) to j) are completed by the following steps:

k) selecting at least one energy ($E$) or one energy range ($\Delta E$);
l) producing an image, each pixel of which comprises a datum obtained from a corrected spectrum, associated with a pixel ($20_{i,j}$) of the detector, at said energy ($E$) or in said energy range ($\Delta E$).

13. Method according to Claim 12, wherein, in step l), each pixel of the image comprises information relating to an integral or to a mean of said corrected spectrum ($S^*_{i,j}$) in said energy range ($\Delta E$).

14. Method according to any one of the preceding claims, wherein the mask (15) is interposed between the radiation source (11) and the object (10).

15. Data storage medium containing instructions for executing steps b) to j) of the method according to any one of Claims 1 to 14, based on the spectra acquired in step a), these instructions being executed by a microprocessor.

16. Device for acquiring spectra of radiation transmitted by an object (10), comprising:

- a radiation source (11) able to emit ionizing electromagnetic radiation, called the incident radiation ($I^0$), toward said object (10);
- a detector (20) comprising pixels ($20_{i,j}$), each pixel being configured to detect radiation (10) transmitted by the object toward the detector, and to acquire a spectrum ($S_{i,j}$) thereof;
- a mask (15), able to be interposed between the source (11) and the object (10), said mask comprising absorbing elements ($15_x$), which are able to absorb a portion of said incident radiation ($I^0$), and a projection of which onto the detector defines shadow zones ($20_x$) that are distant from one another;
- a processor (21), configured to receive the spectra acquired by each pixel, and to implement steps b) to j) of the method that is the subject of any one of Claims 1 to 14.

**Fig. 1A**

**Fig. 1B**

**Fig. 1C**

**Fig. 2A**

**Fig. 2B**

**Fig. 2C**

**Fig. 3A**

$$S_{i,j \notin 20_x} \quad \Big| \quad S_{i,j \in 20_x}$$

110

$$\Omega$$

$$S_\Omega^{diff}$$

120

130

$$S_{i,j \in 20_{x,\Omega}}$$

150

$$S_{i,j \notin 20_{x,\Omega}}$$

$$S_{i,j \notin 20_{x,\Omega}}^{p}$$

$$S_{i,j \in 20_{x,\Omega}}^{p,x}$$

155

$$M'$$

$$S_{i,j \notin 20_{x,\Omega}}^{p,x}$$

165

$$\varepsilon | S_\Omega^{diff}$$

170

$$\widehat{S}_\Omega^{diff}$$

180

190

$$\widehat{S}_{i,j}^{diff}$$

200

$$S_{i,j \notin 20_x}$$

$$S_{i,j \in 20_x}$$

$$\widehat{S}_{i,j \notin 20_x}^{p} \quad \Big| \quad \widehat{S}_{i,j \in 20_x}^{p}$$

**Fig. 3B**

34

**Fig. 4A**

**Fig. 4B**

**Fig. 4C**

**Fig. 5A**

**Fig. 5B**

**Fig. 5C**   **Fig. 5D**   **Fig. 5E**

**Fig. 5F**

**Fig. 6A**

**Fig. 6B**

**Fig. 6C**

**Fig. 6D**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3024235

**Littérature non-brevet citée dans la description**

- **SOSSIN A.** Fast scattering simulation tool for multi-energy x-ray imaging. *Nuclear Instruments and Methods in Physics Research,* 2015, vol. A 802, 60-66
- **ZHU L.** Scatter Correction Method for X-ray CT Using Primary Modulation : Theory and Preliminary Results. *IEEE Transactions on Médical Imaging,* Décembre 2006, vol. 25 (12
- **NING R.** X-ray Scatter Correction Algorithm for Cone Beam CT-Imaging. *Med.Phys.,* Mai 2004, vol. 31 (5
- **YANG K.** A breast-specific, negligible-dose scatter correction technique for dedicated cone-beam breast CT : a physics-based approach to improve Hounsfield Unit accuracy. *Phys.Med.Biol.,* 2014, vol. 59, 6487-6505
- **FELDKAMP L. et al.** Practical cone-beam algorithm. *JOSA A,* 1984, vol. 1 (6), 612-619